# EUROPEAN PATENT APPLICATION

(11) **EP 4 553 485 A1**
(43) Date of publication of application: **14.05.2025**
(21) Application number: 24211029.4
(22) Date of filing: 06.11.2024
(51) Int. Cl.: G01N 15/075, G01N 21/53, G01N 21/85, G01N 15/01, G01N 21/03

(54) **DETERMINATION OF THE CELL CONCENTRATION AND/OR PLATELET MASS INDEX OF A FLUID**

(30) Priority: 08.11.2023 US 202363547710 P; 14.03.2024 US 202463565070 P
(71) Applicant: Fenwal, Inc., Lake Zurich, IL 60047 (US)
(72) Inventor: KUSTERS, Benjamin E., Lake Zurich, 60047 (US); MADSEN, James G., Lake Zurich, 60047 (US); BETANCOURT, Kyle, Lake Zurich, 60047 (US)
(74) Representative: Maikowski & Ninnemann Patentanwälte Partnerschaft mbB

(57) **Abstract**

An optical detection assembly for monitoring a fluid includes a light source, a light detector array, and a controller. The controller receives signals from the light detector array that are indicative of the intensity of light received by the light detector array after the light has passed through the fluid. The controller generates a scattering profile based on the signals, with the scattering profile including rising and falling edges each having a slope. The controller calculates one or both slopes and determines the platelet mass index (for a platelet-containing fluid) and/or the concentration of a substance in the fluid based on said slope(s). The optical detection assembly may also be used to analyze a reference fluid having a known concentration of the substance, with signals received by the controller when analyzing the reference fluid being a factor when determining the concentration of the substance in the fluid of interest.

## Description

### Background

### Field of the Disclosure

The present disclosure relates to optical monitoring of fluids. More particularly, the present disclosure relates to determination of the concentration of one or more substances in a fluid and/or of the platelet mass index of a fluid being optically monitored using a light source and a light detector array.

### Description of Related Art

It is known to employ an optical detection assembly to monitor the flow of blood, blood components, and other biological fluids through a fluid flow circuit to determine various characteristics of the flow. A typical optical detection assembly includes a light source (e.g., a laser or a light-emitting diode) configured to emit light into a fluid-containing vessel of the fluid flow circuit, with a light detector (e.g., a photodiode) configured to receive light exiting the vessel. The light detector transmits a signal to a controller based upon the light it has received, with the controller using the signal to determine one or more properties of the fluid.

A conventional optical detection assembly may have any of a number of possible shortcomings, depending on its exact configuration. For example, it is common for an optical detection assembly to monitor flow of a biological fluid through flexible plastic tubing of a fluid flow circuit. When light is incident upon plastic tubing, the transport of light into the tubing lumen may vary according to Snell's Law depending on the refractive indices of the materials and incident light angles formed by the tubing surface. The refractive index of air (which is approximately 1) and the refractive index of plastic (which may typically be approximately 1.3 to 1.5) are quite different, and when combined with inconsistent formation of the tubing surface from procedure to procedure and, thus, varying incident angles, light transport into the tubing will vary among procedures, leading to inconsistent measurements of fluid properties.

Another possible shortcoming is the configuration of the light detector of a conventional optical detection assembly, which is frequently a single photodiode. By such a configuration, only the amplitude of light exiting the vessel at a single location is known, whereas light transmitted through turbid media (e.g., blood or a blood component) will be dispersed, rather than exiting along a single path that can be fully received by a single photodiode.

U.S. Patent Application Publication No. 2023/0243746 A1 (the disclosure of which is hereby incorporated herein by reference) describes an optical detection assembly that improves upon such conventional optical detection assemblies. The optical detection assembly described in U.S. Patent Application Publication No. 2023/0243746 A1 is based upon the principle that light exiting a turbid media (such as blood or a blood component) will be dispersed, such that the light may be detected at multiple positions using a light detector array, rather than at a single location by a single light detector (e.g., an individual photodiode). Different fluids (e.g., ones having different concentrations of a target substance) result in emerging light beams having different dispersion patterns, with individual light detectors or light-sensing elements of a light detector array receiving various amounts of light that has been transmitted through the fluid. Based on the maximum intensity of light received by one of the individual light detectors, a summation of the intensity of light received by at least two of the individual light detectors, or the width of the dispersion pattern (corresponding to the number of the individual light detectors that have received some minimum amount of light), the controller may determine the concentration of a substance (e.g., platelets) in the subject fluid.

One of the characteristics of a platelet-containing substance (e.g., whole blood or platelet-rich plasma or platelet concentrate) is its platelet mass index or "PMI," which is the units of platelet volume per fluid volume and which may be calculated by multiplying the platelet concentration of the substance and its mean platelet volume or "MPV." One conventional approach to determining the PMI of a fluid is via use of a cell counter, which is an invasive approach that measures the platelet count and platelet size within a sample, with those values then being used to calculate the PMI of the fluid. One practical application of the relationship between PMI, platelet concentration, and MPV is the determination of the volume of a storage solution to use with a platelet product. According to one conventional approach, the operator of a fluid processing system will provide the system with the target platelet yield of a procedure, along with inputting the MPV of the fluid to be mixed with the storage solution (as provided, for example, by a cell counter). Possible disadvantages of this approach are an inaccuracy of the MPV measured by the cell counter and a difference between the target platelet yield and an actual platelet yield, which can result in an inappropriate volume of storage solution being employed.

Accordingly, it would be advantageous to provide an approach to determining the PMI of a fluid that improves upon the conventional approach, which may include providing a non-invasive approach to determination of PMI.

### Summary

There are several aspects of the present subject matter which may be embodied separately or together in the devices and methods described and claimed below. These aspects may be employed alone or in combination with other aspects of the subject matter described herein, and the description of these aspects together is not intended to preclude the use of these aspects separately or the claiming of such aspects separately as set forth in the claims appended hereto.

In one aspect, an optical detection assembly for monitoring a fluid in a vessel includes a light source configured and oriented to emit a light into a fluid in a vessel, a light detector array comprising a plurality of light detectors and configured to receive at least a portion of the light exiting the vessel, and a controller. The controller is programmed to receive signals from the light detector array indicative of the intensity of the portion of the light received by each one of the light detectors and then generate a scattering profile based at least in part on the signals from the light detector array. The controller calculates the slope of a rising edge of the scattering profile and/or the slope of the falling edge of the scattering profile, with one or both slopes being used to determine the platelet mass index and/or the concentration of a substance in the fluid in the vessel.

In another aspect, a biological fluid processing device includes a pump system, a valve system, a controller programmed to control the operation of the pump system and the valve system to execute a biological fluid processing procedure, and an optical detection assembly. The optical detection assembly includes a light source configured and oriented to emit a light into a fluid in a vessel and a light detector array comprising a plurality of light detectors and configured to receive at least a portion of the light exiting the vessel. The controller is further programmed to receive signals from the light detector array indicative of the intensity of the portion of the light received by each one of the light detectors, with a scattering profile being generated based at least in part on the signals from the light detector array. The scattering profile includes a rising edge and a falling edge, with the slope of the rising edge and/or the slope of the falling edge being calculated and then used to determine the platelet mass index and/or the concentration of a substance in the fluid in the vessel.

In yet another aspect, a method of determining the platelet mass index and/or the concentration of a substance in a fluid in a vessel includes emitting a light into a fluid in a vessel and receiving at least a portion of the light exiting the vessel with a plurality of light detectors of a light detector array. A scattering profile is generated, with the scattering profile being based at least in part on the intensity of the portion of the light received by each one of the light detectors. The scattering profile includes a rising edge and a falling edge, with the slope of the rising edge and/or the slope of the falling edge being calculated. The platelet mass index and/or the concentration of a substance in the fluid in the vessel is then determined based at least in part on the slope(s).

In another aspect, an optical detection assembly for monitoring a fluid in a vessel includes a light source assembly configured and oriented to emit a light into a fluid in a vessel, a light detector assembly configured to receive at least a portion of the light exiting the vessel, and a controller. The controller is programmed to receive a first signal from the light detector assembly indicative of the intensity of the portion of the light received by the light detector assembly when a reference fluid is in the vessel and to receive a second signal from the light detector assembly indicative of the intensity of the portion of the light received by the light detector assembly when a subject fluid is in the vessel. The controller compares the first signal and the second signal and determines the concentration of a substance in the subject fluid in the vessel based at least in part on the comparison between the first signal and the second signal.

In yet another aspect, a biological fluid processing device includes a pump system, a valve system, a controller programmed to control the operation of the pump system and the valve system to execute a biological fluid processing procedure, and an optical detection assembly. The optical detection assembly includes a light source assembly configured and oriented to emit a light into a fluid in a vessel and a light detector assembly configured to receive at least a portion of the light exiting the vessel. The controller is further programmed to receive a first signal from the light detector assembly indicative of the intensity of the portion of the light received by the light detector assembly when a reference fluid is in the vessel, with the controller receiving a second signal from the light detector assembly indicative of the intensity of the portion of the light received by the light detector assembly when a subject fluid is in the vessel. The controller compares the first signal and the second signal and determines the concentration of a substance in the subject fluid in the vessel based at least in part on the comparison between the first signal and the second signal.

In another aspect, a method of determining the concentration of a substance in a subject fluid in a vessel includes emitting a first light into a reference fluid in a vessel. At least a portion of the first light exiting the vessel is received with a light detector assembly and a first signal is generated, with the first signal being indicative of the intensity of the portion of the first light received by the light detector assembly. A second light is emitted into a subject fluid in the vessel, with at least a portion of the second light exiting the vessel being received with the light detector assembly, followed by a second signal being generated, with the second signal being indicative of the intensity of the portion of the second light received by the light detector assembly. The first signal is compared to the second signal, with the concentration of a substance in the subject fluid in the vessel being determined based at least in part on the comparison between the first signal and the second signal.

### Brief Description of the Drawings

Fig. 1 is a perspective view of an exemplary hardware component of a biological fluid processing system according to an aspect of the present disclosure;
Figs. 2 and 2A are schematic views of an exemplary disposable component that may be mounted to the hardware component of Fig. 1 to complete a biological fluid processing system according to an aspect of the present disclosure;
Fig. 3 is a perspective view of an exemplary optical detection assembly of the hardware component of Fig. 1, with a lid thereof in an open position;
Fig. 4 is a perspective view of the optical detection assembly of Fig. 3, with the lid in a closed position;
Fig. 5 is a perspective view of selected components of the optical detection assembly of Fig. 3;
Fig. 6 is a diagrammatic view of the optical detection assembly of Fig. 3, monitoring a fluid having a low cellular concentration and PMI;
Fig. 7 is a diagrammatic view of the optical detection assembly of Fig. 3, monitoring a fluid having a high cellular concentration and PMI;
Fig. 8 is a chart illustrating a scattering profile generated using signals from a light detector array of the optical detection assembly of Fig. 3;
Fig. 9 is a chart illustrating a portion of a scattering profile generated using signals from the light detector array of the optical detection assembly of Fig. 3;
Fig. 10 is a chart illustrating an exemplary correlation between the slope of a portion of a scattering profile and the concentration of platelets in a fluid;
Fig. 11 is a diagrammatic view of the optical detection assembly of Fig. 3, monitoring a reference fluid having a known cellular concentration;
Fig. 12 is a diagrammatic view of the optical detection assembly of Fig. 3, monitoring a subject fluid having an unknown cellular concentration;
Figs. 13 and 14 are pairs of charts illustrating scattering profiles generated when monitoring a reference fluid vs. a subject fluid;
Fig. 15 is a flowchart illustrating an approach to determining the cellular concentration of a subject fluid according to an aspect of the present disclosure;
Figs. 16 and 17 illustrate another embodiment of an optical detection assembly according to an aspect of the present disclosure, with a carriage thereof in different positions; and
Fig. 18 is a chart illustrating an exemplary correlation between the slope of a portion of a scattering profile and the PMI of a fluid.

### Description of the Illustrated Embodiments

The embodiments disclosed herein are for the purpose of providing an exemplary description of the present subject matter. They are, however, only exemplary, and the present subject matter may be embodied in various forms. Therefore, specific details disclosed herein are not to be interpreted as limiting the subject matter as defined in the accompanying claims.

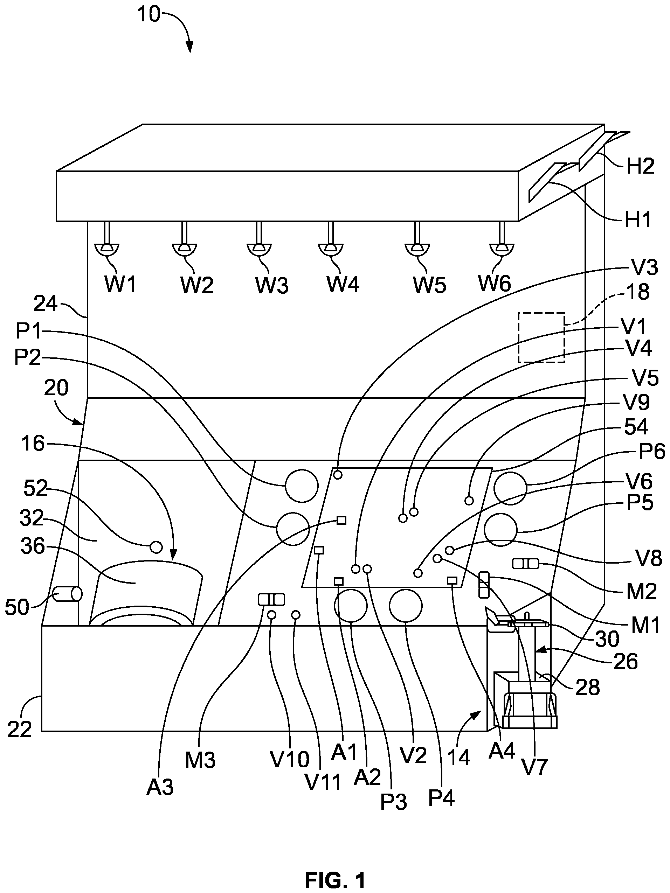

Figs. 1-2A show components of a biological fluid processing system that embodies various aspects of the present subject matter. Use of the system for separating blood into two or more components and collecting at least one of the components will be described herein, though it should be understood that systems according to the present disclosure can be used for processing a variety of different biological fluids.

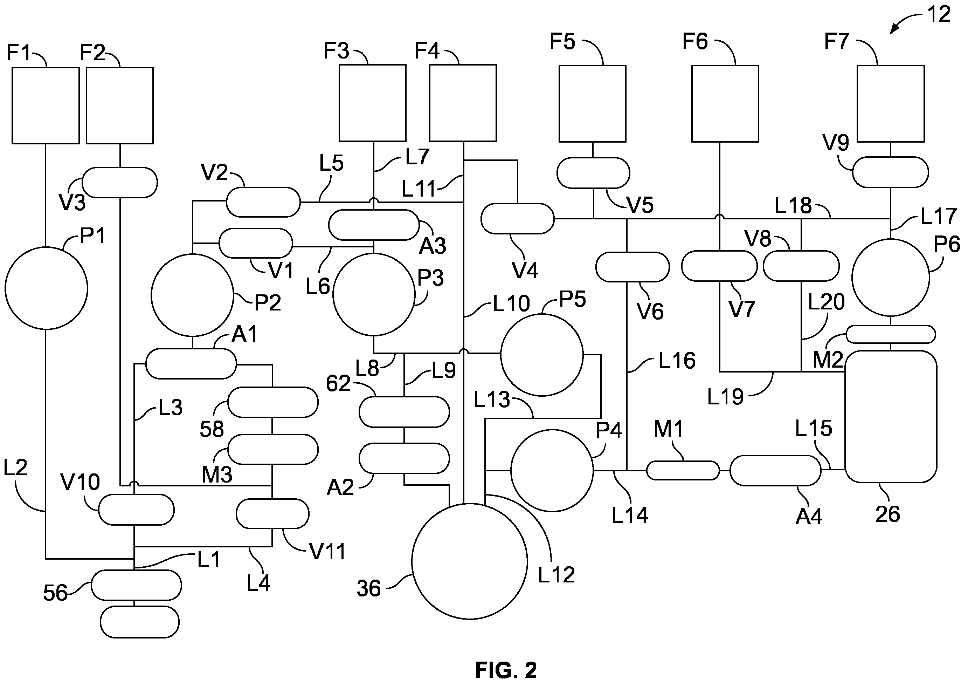

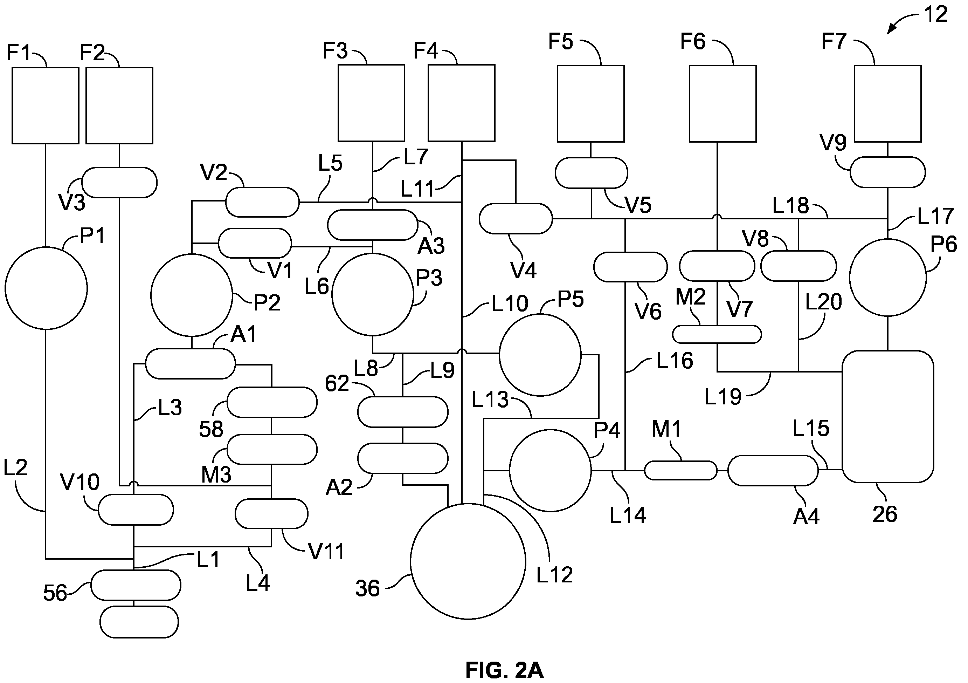

Generally speaking, the system includes two principal components, a durable and reusable biological fluid processing device 10 (Fig. 1) and a disposable fluid flow circuit 12 (Figs. 2 and 2A). The illustrated biological fluid processing device 10 includes a spinning membrane separator drive unit 14, a centrifuge or centrifugal separator 16, additional components that control fluid flow through the disposable fluid flow circuit 12, and a controller 18, which governs the operation of the other components of the biological fluid processing device 10 to perform a biological fluid processing procedure. While the principles described herein may be employed when using the biological fluid processing device 10 of Fig. 1, it should be understood that these same principles may be applied to other biological fluid processing devices, including devices employing single separation technologies or approaches.

### I. The Durable Biological Fluid Processing Device

The biological fluid processing device 10 (Fig. 1) is configured as a durable item that is capable of long-term use. It should be understood that the biological fluid processing device 10 of Fig. 1 is merely exemplary of one possible configuration and that biological fluid processing devices according to the present disclosure may be differently configured.

In the illustrated embodiment, the biological fluid processing device 10 is embodied in a single housing or case 20. The illustrated case 20 includes a generally horizontal portion 22 (which may include an inclined or angled face or upper surface for enhanced visibility and ergonomics) and a generally vertical portion 24. The spinning membrane separator drive unit 14 and the centrifugal separator 16 are shown as being incorporated into the generally horizontal portion 22 of the case 20, while the controller 18 is shown as being incorporated into the generally vertical portion 24.

### A. Spinning Membrane Separator Drive Unit

The biological fluid processing device 10 includes a spinner support or spinning membrane separator drive unit 14 for accommodating a generally cylindrical spinning membrane separator 26 of the fluid flow circuit 12. U.S. Patent No. 5,194,145 (which is hereby incorporated herein by reference) describes an exemplary spinning membrane separator drive unit that would be suitable for incorporation into the fluid processing device 10, but it should be understood that the spinning membrane separator drive unit 14 may be differently configured without departing from the scope of the present disclosure.

The illustrated spinning membrane separator drive unit 14 has a base 28 configured to receive a lower portion of the spinning membrane separator 26 and an upper end cap 30 to receive an upper portion of the spinning membrane separator 26. Preferably, the upper end cap 30 is positioned directly above the base 28 to orient a spinning membrane separator 26 received by the spinning membrane separator drive unit 14 vertically and to define a vertical axis about which the spinning membrane separator 26 is spun. While it may be advantageous for the spinning membrane separator drive unit 14 to vertically orient a spinning membrane separator 26, it is also within the scope of the present disclosure for the spinning membrane separator 26 to be differently oriented when mounted to the biological fluid processing device 10.

In one embodiment, one of the base 28 and upper end cap 30 of the spinning membrane separator drive unit 14 is movable with respect to the other, which may allow differently sized spinning membrane separators 26 to be received by the spinning membrane separator drive unit 14. For example, the upper end cap 30 may be translated vertically with respect to the base 28 and locked in a plurality of different positions, with each locking position corresponding to a differently sized spinning membrane separator 26.

At least one of the base 28 and the upper end cap 30 is configured to spin one or more components of the spinning membrane separator 26 about the axis defined by the spinning membrane separator drive unit 14. The mechanism by which the spinning membrane separator drive unit 14 spins one or more components of the spinning membrane separator 26 may vary without departing from the scope of the present disclosure. In one embodiment, a component of the spinning membrane separator 26 to be spun includes at least one element configured to be acted upon by a magnet (e.g., a metallic material), while the spinning membrane separator drive unit 14 includes a magnet (e.g., a series of magnetic coils or semicircular arcs). By modulating the magnetic field acting upon the aforementioned element of the spinning membrane separator 26, the component or components of the spinning membrane separator 26 may be made to spin in different directions and at varying speeds. In other embodiments, different mechanisms may be employed to spin the component or components of the spinning membrane separator 26.

Regardless of the mechanism by which the spinning membrane separator drive unit 14 spins the component or components of the spinning membrane separator 26, the component or components of the spinning membrane separator 26 is/are preferably spun at a speed that is sufficient to create Taylor vortices in a gap between the spinning component and a stationary component of the spinning membrane separator 26 (or a component that spins at a different speed). Fluid to be separated within the spinning membrane separator 26 flows through this gap, and filtration may be dramatically improved by the creation of Taylor vortices.

### B. Centrifugal Separator

As for the centrifugal separator 16, it includes a centrifuge compartment 32 that receives a centrifugal separation chamber 36 of the fluid flow circuit 12, as well as other components of the centrifugal separator 16. Further details as to the centrifugal separator are set forth in PCT Patent Application Publication No. WO 2018/053217 A1, which is hereby incorporated herein by reference.

Fluid (e.g., anticoagulated whole blood) is introduced into the centrifugal separation chamber 36 by an umbilicus, with the fluid being separated into a layer of less dense components (e.g., platelet-rich plasma, in the case of blood being separated) and a layer of more dense components (e.g., packed red blood cells) within the centrifugal separation chamber 36 as a result of centrifugal forces as it rotates. Components of an interface monitoring system may be positioned within the centrifuge compartment 32 to oversee separation of fluid within the centrifugal separation chamber 36. The interface monitoring system may include a light source 50 and a light detector 52, which is positioned and oriented to receive at least a portion of the light emitted by the light source 50.

The orientation of the various components of the interface monitoring system depends at least in part on the particular configuration of the centrifugal separation chamber 36. In general, though, the light source 50 emits a light beam (e.g., a laser light beam) through the separated fluid components within the centrifugal separation chamber 36 (which may be formed of a material that substantially transmits the light or at least a particular wavelength of the light without absorbing it). A portion of the light reaches the light detector 52, which transmits a signal to the controller 18 that is indicative of the location of an interface between the separated fluid components. If the controller 18 determines that the interface is in the wrong location (which can affect the separation efficiency of the centrifugal separator 16 and/or the quality of the separated fluid components), then it can issue commands to the appropriate components of the biological fluid processing device 10 to modify their operation so as to move the interface to the proper location.

### C. Other Components Of The Biological Fluid Processing Device

In addition to the spinning membrane separator drive unit 14 and the centrifugal separator 16, the biological fluid processing device 10 may include other components compactly arranged to aid fluid processing.

The generally horizontal portion 22 of the case 20 of the illustrated fluid processing device 10 includes a cassette station 54, which accommodates a flow control cassette of the fluid flow circuit 12. In one embodiment, the cassette station 54 is similarly configured to the cassette station of U.S. Patent No. 5,868,696 (which is hereby incorporated herein by reference), but is adapted to include additional components and functionality. The illustrated cassette station 54 includes a plurality of clamps or valves V1-V9 (which are collectively referred to herein as the "valve system" of the biological fluid processing system 10), which move between a plurality of positions (e.g., between a retracted or lowered position and an actuated or raised position) to selectively contact or otherwise interact with corresponding valve stations of the flow control cassette of the fluid flow circuit 12. Depending on the configuration of the fluid flow circuit 12, its cassette may not include a valve station for each valve V1-V9 of the cassette station 54, in which case fewer than all of the valves V1-V9 will be used in a fluid processing procedure.

In the actuated position, a valve V1-V9 engages the associated valve station to prevent fluid flow through that valve station (e.g., by closing one or more ports associated with the valve station, thereby preventing fluid flow through that port or ports). In the retracted position, a valve V1-V9 is disengaged from the associated valve station (or less forcefully contacts the associated valve station than when in the actuated position) to allow fluid flow through that valve station (e.g., by opening one or more ports associated with the valve station, thereby allowing fluid flow through that port or ports). Additional clamps or valves V10 and V11 of the valve system may be positioned outside of the cassette station 54 to interact with portions of valve stations (which may be lengths of tubing) of the fluid flow circuit 12 to selectively allow and prevent fluid flow therethrough. The valves V1-V9 and corresponding valve stations of the cassette station 54 and cassette may be differently configured and operate differently from the valves V10 and V11 and the valve stations that are spaced away from the cassette station 54.

The cassette station 54 may be provided with additional components, such as pressure sensors A1-A4, which interact with sensor stations of the cassette to monitor the pressure at various locations of the fluid flow circuit 12. For example, if the fluid source is a human donor, one or more of the pressure sensors A1-A4 may be configured to monitor the pressure of the donor's vein during blood draw and return. Other pressure sensors A1-A4 may monitor the pressure of the spinning membrane separator 26 and the centrifugal separation chamber 36. The controller 18 may receive signals from the pressure sensors A1-A4 that are indicative of the pressure within the fluid flow circuit 12 and, if a signal indicates a low- or highpressure condition, the controller 18 may initiate an alarm or error condition to alert an operator to the condition and/or to attempt to bring the pressure to an acceptable level without operator intervention.

The biological fluid processing device 10 may also include a plurality of pumps P1-P6 (which are collectively referred to herein as the "pump system" of the biological fluid processing device 10) to cause fluid to flow through the fluid flow circuit 12. The pumps P1-P6 may be differently or similarly configured and/or function similarly or differently from each other. In the illustrated embodiment, the pumps P1-P6 are configured as peristaltic pumps, which may be generally configured as described in U.S. Patent No. 5,868,696. Each pump P1-P6 engages a different tubing loop extending from a side surface of the flow control cassette and may be selectively operated under command of the controller 18 to cause fluid to flow through a portion of the fluid flow circuit 12. In one embodiment, all or a portion of the cassette station 54 may be capable of translational motion in and out of the case 20 to allow for automatic loading of the tubing loops into the associated pump P1-P6. In another exemplary embodiment, rather than employing peristaltic pumps, pneumatic pumps may be employed, with actuators incorporated into the cassette station 54 interacting with suitably configured portions of the fluid flow circuit 12 (e.g., pump stations of a cassette mounted to the cassette station 54) to convey fluid through the fluid flow circuit 12.

The illustrated biological fluid processing device 10 also includes a spinner inlet sensor M1 for determining one or more properties of a fluid flowing into a spinning membrane separator 26 mounted within the spinning membrane separator drive unit 14. If the fluid flowing into the spinning membrane separator 26 is whole blood (which may include anticoagulated whole blood), the spinner inlet sensor M1 may be configured to determine the hematocrit of the blood flowing into the spinning membrane separator 26. If the fluid flowing into the spinning membrane separator 26 is platelet-rich plasma, the spinner inlet sensor M1 may be configured to determine the platelet concentration and/or PMI of platelet-rich plasma flowing into the spinning membrane separator 26. The spinner inlet sensor M1 may detect the one or more properties of a fluid by optically monitoring the fluid as it flows through tubing of the fluid flow circuit 12, or by any other suitable approach. The controller 18 may receive signals from the spinner inlet sensor M1 that are indicative of the one or more properties of fluid flowing into the spinning membrane separator 26 and use the signals to optimize the fluid processing procedure based upon that property or properties. If the property or properties is/are outside of an acceptable range, then the controller 18 may initiate an alarm or error condition to alert an operator to the condition. A suitable device and method for monitoring hematocrit and/or platelet concentration is described in U.S. Patent No. 6,419,822 (which is hereby incorporated herein by reference), but it should be understood that a different approach may also be employed for monitoring one or more properties of a fluid or fluid component flowing into the spinning membrane separator 26.

The illustrated biological fluid processing device 10 further includes a spinner outlet sensor M2, which accommodates tubing of the fluid flow circuit 12 that flows a separated fluid component out of the spinning membrane separator 26. The spinner outlet sensor M2 monitors the separated fluid component to determine one or more properties thereof, and may do so by optically monitoring the separated fluid component as it flows through the tubing or by any other suitable approach. In one embodiment (which is illustrated in Fig. 2), separated plasma flows through the tubing, in which case the spinner outlet sensor M2 may be configured to determine the amount of cellular blood components in the plasma and/or whether the plasma is hemolytic and/or lipemic. This may be done using an optical monitor of the type described in U.S. Patent No. 8,556,793 (which is hereby incorporated herein by reference) that measures the optical density of the fluid in the associated tubing, or by any other suitable device and/or method. In another embodiment (which is illustrated in Fig. 2A), the spinner outlet sensor M2 is instead associated with tubing that receives a flow of separated platelets or platelet concentrate exiting the spinning membrane separator 26, in which case the spinner outlet sensor M2 may be configured to determine its PMI, as will be described in greater detail.

The illustrated fluid processing device also includes an air detector M3 (e.g., an ultrasonic bubble detector), which accommodates tubing of the fluid flow circuit 12 that flows fluid to a recipient. It may be advantageous to prevent air from reaching the recipient, whether a human recipient (e.g., the same human that serves as the blood source) or a non-human recipient (e.g., a storage bag or container), so the air detector M3 may transmit signals to the controller 18 that are indicative of the presence or absence of air in the tubing. If the signal is indicative of air being present in the tubing, the controller 18 may initiate an alarm or error condition to alert an operator to the condition and/or to take corrective action to prevent the air from reaching the recipient (e.g., by reversing the flow of fluid through the tubing or diverting flow to a vent location).

The generally vertical portion 24 of the case 20 may include a plurality of volume measurement systems W1-W6 (six are shown, but more or fewer may be provided), each configured to be associated with one or more fluid containers F1-F7 of the fluid flow circuit 12 (Figs. 2 and 2A). Each volume measurement system W1-W6 is configured to work in combination with the controller 18 to measure a current volume of fluid within an associated fluid container F1-F7 and to calculate a change in that volume between two or more points in time. The individual volume measurement systems W1-W6 may be variously configured without departing from the scope of the present disclosure, which may include two or more of the volume measurement systems W1-W6 being differently configured. In one exemplary embodiment, a volume measurement system W1-W6 may be configured as or include a weight scale configured to support and measure the weight of a fluid within an associated fluid container F1-F7 (with the measured weight being converted to a volume by a component of the volume measurement system W1-W6 or by the controller 18). In another exemplary embodiment, a volume measurement system W1-W6 may include one or more sensors configured to detect a volume and/or a change in volume of a fluid within an associated fluid container F1-F7. Volume measurement systems including additional components (e.g., both a weight scale and a sensor) and/or alternative components may also be employed without departing from the scope of the present disclosure.

Regardless of its particular configuration, each volume measurement system W1-W6 transmits to the controller 18 a signal that is indicative of the volume of the fluid within the associated container F1-F7 to track the change of volume during the course of a procedure. This allows the controller 18 to process the incremental volume changes to derive fluid processing volumes and flow rates and subsequently generate signals to control processing events based, at least in part, upon the derived processing volumes. For example, the controller 18 may diagnose leaks and obstructions in the fluid flow circuit 12 and alert an operator.

The illustrated case 20 is also provided with a plurality of hooks or supports H1 and H2 that may support various components of the fluid flow circuit 12 or other suitably sized and configured objects.

### D. Controller

According to an aspect of the present disclosure, the biological fluid processing device 10 includes a controller 18, which is suitably configured and/or programmed to control operation of the biological fluid processing device 10. In one embodiment, the controller 18 comprises a main processing unit (MPU), which can comprise, e.g., a Pentium^{™} type microprocessor made by Intel Corporation, although other types of conventional microprocessors can be used. In one embodiment, the controller 18 may be mounted inside the generally vertical portion 24 of the case 20, adjacent to or incorporated into an operator interface station (e.g., a touchscreen). In other embodiments, the controller 18 and operator interface station may be associated with the generally horizontal portion 22 or may be incorporated into a separate device that is connected (either physically, by a cable or the like, or wirelessly) to the biological fluid processing device 10.

The controller 18 is configured and/or programmed to execute at least one biological fluid processing procedure but, more advantageously, is configured and/or programmed to execute a variety of different biological fluid processing procedures. For example, the controller 18 may be configured and/or programmed to carry out one or more of the following: a double unit red blood cell collection procedure, a plasma collection procedure, a plasma/red blood cell collection procedure, a red blood cell/platelet/plasma collection procedure, a platelet collection procedure, and a platelet/plasma collection procedure.

More particularly, in carrying out these fluid processing procedures, the controller 18 is configured and/or programmed to control one or more of the following tasks: drawing fluid into a fluid flow circuit 12 mounted to the biological fluid processing device 10, conveying fluid through the fluid flow circuit 12 to a location for separation (i.e., into a spinning membrane separator 26 or centrifugal separation chamber 36 of the fluid flow circuit 12), separating the fluid into two or more components as desired, and conveying the separated components into storage containers, to a second location for further separation (e.g., into whichever of the spinning membrane separator 26 and centrifugal separation chamber 36 that was not used in the initial separation stage), or to a recipient (which may be a source from which the fluid was originally drawn).

This may include instructing the spinning membrane separator drive unit 14 and/or the centrifugal separator 16 to operate at a particular rotational speed and instructing a pump P1-P6 to convey fluid through a portion of the fluid flow circuit 12 at a particular flow rate. Hence, while it may be described herein that a particular component of the biological fluid processing device 10 (e.g., the spinning membrane separator drive unit 14 or the centrifugal separator 16) performs a particular function, it should be understood that that component is being controlled by the controller 18 to perform that function.

Before, during, and after a procedure, the controller 18 may receive signals from various components of the biological fluid processing device 10 (e.g., the pressure sensors A1-A4) to monitor various aspects of the operation of the biological fluid processing device 10 and characteristics of the fluid and separated fluid components as they flow through the fluid flow circuit 12. If the operation of any of the components and/or one or more characteristics of the fluid or separated fluid components is outside of an acceptable range, then the controller 18 may initiate an alarm or error condition to alert the operator and/or take action to attempt to correct the condition. The appropriate corrective action will depend upon the particular error condition and may include action that is carried out with or without the involvement of an operator.

For example, the controller 18 may include an interface control module, which receives signals from the light detector 52 of the interface monitoring system. The signals that the controller 18 receives from the light detector 52 are indicative of the location of an interface between the separated fluid components within the centrifugal separation chamber 36. If the controller 18 determines that the interface is in the wrong location, then it can issue commands to the appropriate components of the biological fluid processing device 10 to modify their operation so as to move the interface to the proper location. For example, the controller 18 may instruct one of the pumps P1-P6 to cause fluid to flow into the centrifugal separation chamber 36 at a different rate and/or for a separated fluid component to be removed from the centrifugal separation chamber 36 at a different rate and/or for the centrifugal separation chamber 36 to be spun at a different speed by the centrifugal separator 16.

If provided, an operator interface station associated with the controller 18 allows the operator to view on a screen or display (in alpha-numeric format and/or as graphical images) information regarding the operation of the system. The operator interface station also allows the operator to select applications to be executed by the controller 18, as well as to change certain functions and performance criteria of the system. If configured as a touchscreen, the screen of the operator interface station can receive input from an operator via touch-activation. Otherwise, if the screen is not a touchscreen, then the operator interface station may receive input from an operator via a separate input device, such as a computer mouse or keyboard. It is also within the scope of the present disclosure for the operator interface station to receive input from both a touchscreen and a separate input device, such as a keypad.

### II. The Disposable Fluid Flow Circuit

As for the fluid flow circuit or flow set 12 (Figs. 2 and 2A), it is intended to be a sterile, single use, disposable item. Before beginning a given fluid processing procedure, the operator loads various components of the fluid flow circuit 12 in the case 20 in association with the biological fluid processing device 10. The controller 18 implements the procedure based upon preset protocols, taking into account other input from the operator. Upon completing the procedure, the operator removes the fluid flow circuit 12 from association with the biological fluid processing device 10. The portions of the fluid flow circuit 12 holding the collected fluid component or components (e.g., collection containers or bags) are removed from the case 20 and retained for storage, transfusion, or further processing. The remainder of the fluid flow circuit 12 is removed from the case 20 and discarded.

In the illustrated embodiment, the fluid flow circuit 12 includes a cassette, to which the other components of the fluid flow circuit 12 are connected by flexible tubing. The other components may include a plurality of fluid containers F1-F7. In the context of the present disclosure these containers include an anticoagulant container F1, a saline container F2, an in-process container F3, a return container F4, a plasma collection container F5, a platelet collection container F6, and an (optional) additive container F7. The illustrated flow circuit 12 further includes one or more fluid source access devices (e.g., a connector for accessing blood within a fluid container or a phlebotomy needle), a spinning membrane separator 26 and a centrifugal separation chamber 36.

The flow control cassette provides a centralized, programmable, integrated platform for all the pumping and many of the valving functions required for a given fluid processing procedure. In one embodiment, the cassette is similarly configured to the cassette of U.S. Patent No. 5,868,696, but is adapted to include additional components (e.g., more tubing loops) and functionality.

In use, the cassette is mounted to the cassette station 54 of the biological fluid processing device 10 so as to align each sensor station with an associated pressure sensor A1-A4 of the cassette station 54 and its valve stations with an associated valve V1-V9. Each valve station may define one or more ports that allow fluid communication between the valve station and another interior cavity of the cassette (e.g., a flow path). As described above, each valve V1-V9 is movable under command of the controller 18 to move between a plurality of positions (e.g., between a retracted or lowered position and an actuated or raised position) to selectively contact the valve stations of the cassette. In the actuated position, a valve V1-V9 engages the associated valve station to close one or more of its ports to prevent fluid flow therethrough. In the retracted position, a valve V1-V9 is disengaged from the associated valve station (or less forcefully contacts the associated valve station than when in the actuated position) to open one or more ports associated with the valve station, thereby allowing fluid flow therethrough.

A plurality of tubing loops extend from the side surface of the cassette to interact with pumps P1-P6 of the biological fluid processing device 10. The different pumps P1-P6 may interact with the tubing loops of the cassette to perform different tasks during a procedure, but in the context of the present disclosure, a different one of the pumps P1-P6 may be configured to serve as an anticoagulant pump P1, a source pump P2, a centrifuge pump P3, an outlet pump P4, a recirculation pump P5, and a plasma pump P6. If the pumps P1-P6 are differently configured (e.g., if they are configured as pneumatic pumps), then the cassette may be differently configured (e.g., with pump stations aligned with pneumatic pump actuators) to allow for the pumps P1-P6 to convey fluid through the cassette.

Additional tubing extends from the side surface of the cassette to connect to the other components of the fluid flow circuit 12, such as the various fluid containers F1-F7, the spinning membrane separator 26, and the centrifugal separation chamber 36. The tubing connected to the centrifugal separator chamber 36 (which includes one inlet tube and two outlet tubes) may be aggregated into an umbilicus.

Various additional components may be incorporated into the tubing leading out of the cassette or into one of the cavities of the cassette. For example, a manual clamp 56 may be associated with a line or lines leading to the fluid source, a return line filter 58 (e.g., a microaggregate filter) may be associated with a line leading to a fluid recipient, and/or an air trap 62 may be positioned on a line upstream of the centrifugal separation chamber 36.

### III. Exemplary Biological Fluid Processing Procedure

An exemplary biological fluid processing procedure according to the present disclosure will now be described.

Prior to processing, an operator selects the desired protocol (e.g., using an operator interface station, if provided), which informs the controller 18 of the manner in which it is to control the other components of the biological fluid processing device 10 during the procedure. This may include first selecting one of a plurality of possible procedures that the system is capable of executing and then, after selecting the nature of the procedure, selecting one or more parameters to be in effect during the procedure. For example, this may include selecting a platelet collection procedure from among a variety of blood separation procedures and then selecting a total volume of blood to be processed or a target volume of platelets to be collected during the procedure. If the fluid source is a living source (e.g., a donor or patient), the operator may proceed to enter various parameters, such as the sex/height/weight of the source. In one embodiment, the operator may also enter one or more characteristics of the fluid to be processed, such as a platelet pre-count.

Once the controller 18 has received the necessary input, it may proceed to instruct the operator to mount the fluid flow circuit 12 to the biological fluid processing device 10. If there are any fluid containers (e.g., a platelet additive solution container) that are not integrally formed with the fluid flow circuit 12, they may be connected to the fluid flow circuit 12 (e.g., by piercing a septum of a tube of the fluid flow circuit 12 or via a luer connector), with the fluid flow circuit 12 then being mounted to the biological fluid processing device 10 (including the fluid containers F1-F7 being associated with the volume measurement systems W1-W6, as appropriate). In one exemplary embodiment, each volume measurement system W1-W6 includes a weight scale associated with a hook from which a fluid container may be hung. In another exemplary embodiment, at least one of the volume measurement systems W1-W6 includes a weight scale associated with a horizontal platform or surface, with a container being placed onto the platform or surface for support while the weight scale sends signals indicative of the weight of the container (and its contents) to be sent to the controller 18 throughout the course of a procedure. In other embodiments, a fluid container may be associated with a volume measurement system omitting a weight scale, but including other means for measuring the volume of fluid within the container (e.g., one or more sensors).

Once the fluid flow circuit 12 has been fully mounted to the biological fluid processing device 10, the controller 18 may proceed with an integrity check of the fluid flow circuit 12 to ensure that the various components of the fluid flow circuit 12 are properly connected and functioning. Following a successful integrity check, the fluid source is connected to the fluid flow circuit 12 (e.g., by connecting to a container of previously collected fluid or by phlebotomizing a donor), and the fluid flow circuit 12 may be primed (e.g., using saline pumped from a saline container F2 by operation of one or more of the pumps P1-P6 of the biological fluid processing device 10).

After the fluid flow circuit 12 has been primed, fluid processing may begin. In a first phase of an exemplary platelet collection procedure, blood is drawn into the fluid flow circuit 12 from a blood source. If the blood source is a donor, then blood may be drawn into the fluid flow circuit 12 through a single needle that is connected to the cassette by line L1. Line L1 may include a manual clamp 56 that may initially be in a closed position to prevent fluid flow through line L1. When processing is to begin, an operator may move the manual clamp 56 from its closed position to an open position to allow fluid flow through line L1.

The blood is drawn into line L1 by the source pump P2 of the biological fluid processing device 10. Anticoagulant from the anticoagulant container F1 may be drawn through line L2 under action of the anticoagulant pump P1 and added to the blood at a junction of lines L1 and L2.

In the illustrated embodiment, valve V10 is open to allow anticoagulated blood to flow through line L3 and a cassette sensor station associated with pressure sensor A1, while valve V11 is closed to prevent fluid flow through line L4. If the blood source is a living body (e.g., a donor), the pressure sensor A1 may communicate with the controller 18 to monitor the pressure within the vein of the blood source.

The cassette includes two valve stations downstream of the source pump P2, with valve V2 being closed to prevent flow through line L5 and valve V1 being open to allow flow through line L6. A portion of the blood is directed through line L7 and a cassette sensor station associated with pressure sensor A3 to the in-process container F3 and the remainder is directed through line L8 toward the centrifuge pump P3, which controls the amount of blood that is directed to the centrifugal separation chamber 36 instead of the in-process container F3. In particular, the flow rate of the source pump P2 is greater than the flow rate of the centrifuge pump P3, with the difference therebetween being equal to the flow rate of blood into the in-process container F3. The flow rates may be selected such that the in-process container F3 is partially or entirely filled with blood at the end of the draw phase.

The blood pumped through line L8 by the centrifuge pump P3 passes through line L19, an air trap 62, and a cassette sensor station associated with pressure sensor A2 (which works in combination with the controller 18 of the biological fluid processing device 10 to monitor the pressure in the centrifugal separation chamber 36) before reaching the centrifugal separation chamber 36 of the fluid flow circuit 12. The centrifugal separator 16 of the biological fluid processing device 10 manipulates the centrifugal separation chamber 36 to separate the blood in the centrifugal separation chamber 36 into platelet-rich plasma and packed red blood cells. In one embodiment, the centrifugal separation chamber 36 is rotated nominally at 4,500 rpm, but the particular rotational speed may vary depending on the flow rates of fluids into and out of the centrifugal separation chamber 36.

The packed red blood cells exit the centrifugal separation chamber 36 via line L10 and flow through line L11 into the return container F4. Platelet-rich plasma is drawn out of the centrifugal separation chamber 36 via line L12 by the combined operation of the recirculation and outlet pumps P5 and P4 of the biological fluid processing device 10. The platelet-rich plasma travels through line L12 until it reaches a junction, which splits into lines L13 and L14. The recirculation pump P5 is associated with line L13 and redirects a portion of the platelet-rich plasma to a junction, where it mixes with blood in line L8 that is being conveyed into the centrifugal separation chamber 36 by the centrifuge pump P3. Recirculating a portion of the platelet-rich plasma into the centrifugal separation chamber 36 with inflowing blood decreases the hematocrit of the blood entering the centrifugal separation chamber 36, which may improve separation efficiency. By such an arrangement, the flow rate of the fluid entering the centrifugal separation chamber 36 is equal to the sum of the flow rates of the centrifuge pump P3 and the recirculation pump P5. As the platelet-rich plasma drawn out of the centrifugal separation chamber 36 into line L13 by the recirculation pump P5 is immediately added back into the centrifugal separation chamber 36, the bulk or net platelet-rich plasma flow rate out of the centrifugal separation chamber 36 is equal to the flow rate of the outlet pump P4.

Line L14 ends at a junction, where it joins with lines L15 and L16. Valve V6 is closed to prevent fluid flow through line L16, thereby directing the separated platelet-rich plasma to the spinning membrane separator 26 via line L15. Before reaching the spinning membrane separator 26, the portion of the platelet-rich plasma conveyed through line L15 passes the spinner inlet sensor M1 and a cassette sensor station associated with pressure sensor A4. The spinner inlet sensor M1 may detect the PMI or the concentration of platelets in the platelet-rich plasma entering the spinning membrane separator 26, while the pressure sensor A4 may monitor the pressure of the spinning membrane separator 26.

While valve V6 is typically closed, it may be selectively opened to divert all or a portion of the platelet-rich plasma from line L14 into and through line L16 and to the return container F4, if necessary. An example would be at the start of a procedure when separation is initializing and platelets are not yet exiting the centrifugal separation chamber 36, in which case the fluid conveyed through line L14 by the outlet pump P4 could be diverted to the return container F4.

The spinning membrane separator drive unit 14 of the biological fluid processing device 10 manipulates the spinning membrane separator 26 to separate the platelet-rich plasma into platelet-poor plasma ("plasma") and platelet concentrate ("platelets"). Plasma is pumped out of the spinning membrane separator 26 via line L17 by the plasma pump P6 of the biological fluid processing device 10. Valves V5, V6, V8, and V9 are closed to direct the separated plasma along line L18, through valve V4, and into the return container F4 (with the separated red blood cells). In the orientation of Fig. 2, on the way to the return container F4, the plasma passes through spinner outlet sensor M2, which may cooperate with the controller 18 to determine one or more characteristics of the plasma, such as the amount of cellular blood components in the plasma and/or whether the plasma is hemolytic and/or lipemic.

The platelet concentrate is conveyed out of the spinning membrane separator 26 via line L19. There is no pump associated with line L19, so instead the flow rate at which the platelets exit the spinning membrane separator 26 is equal to the difference between the flow rates of the outlet pump P4 and plasma pump P6. Valve V8 is closed to prevent fluid flow through line L20, thereby directing the flow of platelets along line L19, through valve V7, and into the platelet collection container F6. In the orientation of Fig. 2A, on the way to the platelet collection container F6, the platelets pass through spinner outlet sensor M2, which may cooperate with the controller 18 to determine one or more characteristics of the platelets, such as the PMI and/or platelet concentration. Valve V8 may be selectively opened to allow fluid flow through line L20 and to a junction, where it joins the plasma flowing through line L18 to the return container F4, if necessary.

Depending on the volume of platelets to be collected, the above-described draw stage may be repeated, with draw stages being alternated with return stages in which blood from the in-process container F3 is separated in the centrifugal separation chamber 36 while previously collected blood components in the return container F4 are returned to the blood source. During such return stages, the separated red blood cells and platelet-rich plasma may be variously routed through the fluid flow circuit 12, typically with an additional volume of platelets being collected in the platelet collection container F6 after being separated from platelet-poor plasma in the spinning membrane separator 26 (as during the draw stage). A platelet additive solution from the additive container F7 may be added to the collected platelets in the platelet collection container F6 before ending the procedure.

### IV. Determination Of Cell Concentration

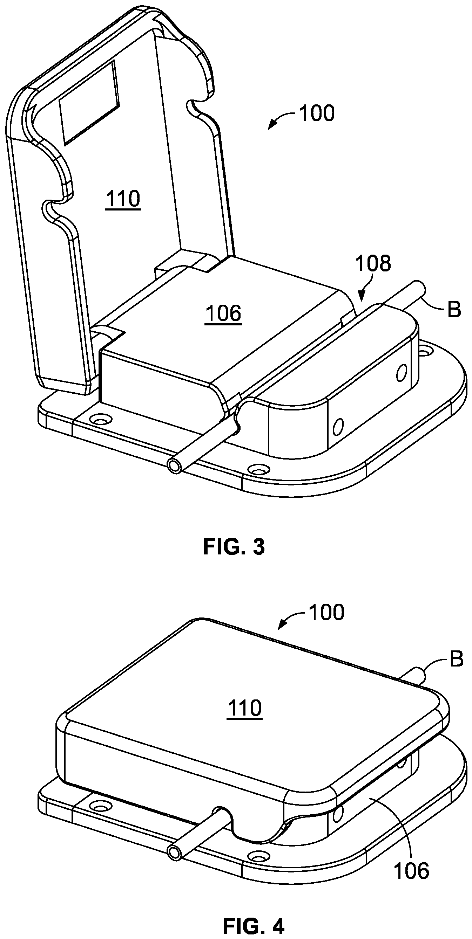

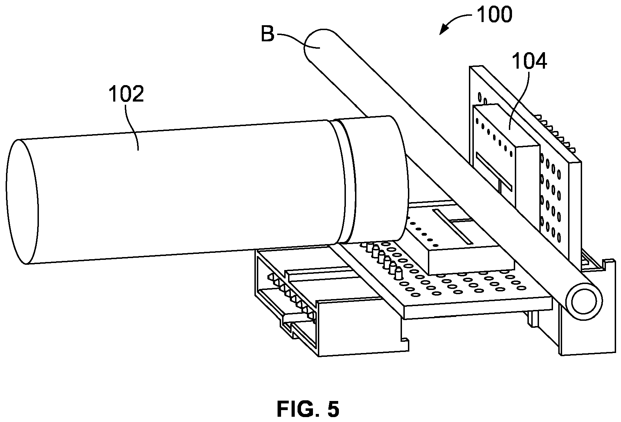

As noted above, the spinner inlet sensor M1 may be used in combination with the controller 18 to determine one or more properties of a fluid flowing into a spinning membrane separator 26, while the spinner outlet sensor M2 may be used to determine one or more properties of a fluid flowing out of the spinning membrane separator 26. Figs. 3-5 illustrate an exemplary optical detection assembly 100 that may be incorporated into the biological fluid processing device 10 to perform the functions of the spinner inlet sensor M1 or the spinner outlet sensor M2. In one embodiment, two such optical detection assemblies 100 may be incorporated into the biological fluid processing device 10, with one acting as the spinner inlet sensor M1 and the other acting as the spinner outlet sensor M2. While the optical detection assembly 100 of Figs. 3-5 will be described herein as being a component of the biological fluid processing device 10 of Fig. 1, it should be understood that optical detection assemblies according to the present disclosure may be incorporated into differently configured biological fluid processing devices or be provided as standalone devices that are not incorporated into a biological fluid processing device.

In the illustrated embodiment, the optical detection assembly 100 includes a light source 102 and a light detector array 104, which are spaced apart to accommodate a vessel "B" therebetween. When the optical detection assembly 100 is employed as a spinner inlet sensor M1, the vessel B may be line L15 of the fluid flow circuit 12, with the vessel B being line L17 (Fig. 2) or line L19 (Fig. 2A) of the fluid flow circuit 12 when the optical detection assembly 100 is instead employed as a spinner outlet sensor M2. It should be understood that the configuration of the vessel B used in combination with the optical detection assembly 100 may vary without departing from the scope of the present disclosure, provided that the vessel B is suitable for containing a fluid (which may include the vessel B being configured to accommodate the flow of a fluid therethrough) and formed of a material that is configured to transmit light emitted by the light source 102.

The illustrated optical detection assembly 100 includes a base 106 defining a slot or channel 108 configured to receive the vessel B. The channel 108 is configured to secure the vessel B in a desired orientation with respect to the light source 102 and the light detector array 104. The optical detection assembly 100 may also include a lid 110 (which is shown in Figs. 3 and 4 as being hingedly or pivotally associated to the base 106) to block external light from interfering with analysis of a fluid within the vessel B.

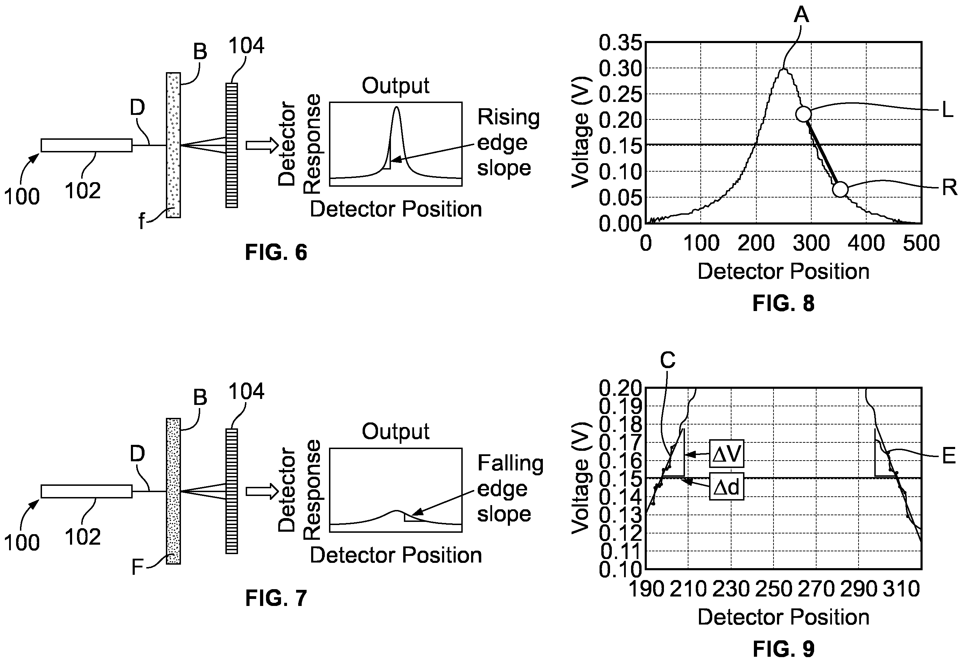

Light D emitted by the light source 102 (which may be variously configured without departing from the scope of the present disclosure) enters and then exits the vessel B after passing through a fluid within the vessel B. Figs. 6 and 7 illustrate transmitted light exiting the vessel B, with the light detector array 104 positioned and oriented to receive at least a portion of the transmitted light. The light detector array 104 is comprised of a plurality of light detectors or light-sensing elements (e.g., 256 photodiodes in a linear array). As explained above, light exiting a turbid media (such as blood or a blood component) will be dispersed, such that the light may be detected at multiple positions, rather than at a single location by a single light detector (e.g., an individual photodiode). It has been found that different fluids (e.g., ones having different concentrations of a target substance) may result in emerging light beams having different dispersion patterns. For example, Fig. 6 illustrates a fluid "f" having a relatively low concentration of platelets, while Fig. 7 illustrates a fluid "F" having a higher concentration of platelets. The light D (which may be a narrowly focused laser beam, for example) transmitted through the fluid in the vessel B is dispersed, with different individual light detectors receiving different portions of the transmitted light.

A controller associated with the light detector array 104 (which may be the controller 18 of the biological fluid processing device 10 or a different, dedicated controller) receives signals from each of the individual light detectors of the light detector array 104, with each signal being indicative of the intensity of light received by the individual light detector that transmitted the signal to the controller. Figs. 6 and 7 include charts generated by the controller based on the signals from the light detector array 104 (which are referred to herein as "scattering profiles") that reflect the intensity of the signal received by the controller from each individual light detector, with the results ordered by the relative positions of the individual light detectors (i.e., the signal from the light detector at the left end of the light detector array 104 is presented at the left end of each chart, with the signal from the adjacent light detector being presented just to the right of the signal from the first light detector and so on until the signal from the light detector at the right end of the light detector array 104 is presented at the right end of each chart). As can be seen in Figs. 6 and 7, the light detectors at the center of the light detector array 104 will tend to receive the most intense light, with the light detectors at each end of the light detector array 104 receiving little to no light.

In the illustrated embodiment, platelets within the fluid cause light to scatter, rather than being transmitted straight through the fluid and vessel B (along its initial path). As there are more cells in the fluid F of Fig. 7, there is more scattering of the light, with more individual light detectors receiving at least some of the light, though with a relatively low maximum intensity compared to the maximum intensity of the light received by an individual light detector in Fig. 6. Stated differently, light passing through the less concentrated fluid f of Fig. 6 is narrowly distributed or dispersed, while light passing through the more highly concentrated fluid F of Fig. 7 is more widely or broadly distributed or dispersed. Thus, by providing a light detector array 104, the intensity of light received by multiple individual light detectors (i.e., the light distribution or the scattering profile) may be assessed to determine one or more properties of a subject fluid, such as a concentration of a substance (e.g., platelets) in the fluid. This may allow for improved measurement accuracy compared to conventional optical detection assemblies having a single light detector and a controller configured only to assess the intensity of light received by the single light detector.

Once the controller has generated a scattering profile, it may employ various approaches to extract data from the scattering profile that can be used to determine a characteristic of a subject fluid. For example, a scattering profile will have an apex at the location corresponding to the individual light detector that has received the most light that has passed through the vessel B and the fluid within the vessel B. The scattering profile will have a "rising edge" to the left of the apex and a "falling edge" to the right of the apex. The rising edge encompasses signals from the individual light detectors to the left of the central light detectors, while the falling edge encompasses signals from the individual light detectors to the right of the central light detectors. As explained above, the light detectors closer to the center of the light detector array 104 will tend to receive more light than the light detectors spaced farther from the center, such that the rising edge will have a positive slope (which will tend to be different at different points along the scattering profile) and the falling edge will have a negative slope (which will tend to be different at different points along the scattering profile).

As the rising edge slope and the falling edge slope will tend to vary at different points along the scattering profile, the controller may employ different techniques to calculate the slope of a section of the rising edge or the falling edge. For example, Fig. 6 illustrates a section of the rising edge of a scattering profile that may be analyzed to calculate a rising edge slope, while Fig. 7 illustrates a section of the falling edge of a scattering profile that may be analyzed to calculate a falling edge slope. In one exemplary approach, the controller selects two points of the rising edge or two points of the falling edge of a scattering profile, corresponding to the signals from two individual light detectors of the light detector array 104. The controller then divides the change in detector response (the voltage of the signal transmitted by the leftmost of the two detectors subtracted from the voltage of the signal transmitted by the rightmost of the two detectors) by the change in detector position (the difference between the relative positions of the two detectors within the light detector array 104) to calculate the slope of the edge. In the example shown in Fig. 8, the falling edge of the scattering profile is analyzed by the controller to calculate its slope, with the signal "L" from the leftmost of the two selected detectors (which has a detector position of 290) having a voltage of 2.1 dV and the signal "R" from the rightmost of the two selected detectors (which has a detector position of 350) having a voltage of 0.7 dV. In this example, the falling edge slope is approximately -0.023 ((0.7 dV - 2.1 dV) / (350 - 290)).

According to another approach, the controller may employ a linear regression to calculate the rising edge slope or the falling edge slope, with Fig. 9 showing regression lines for calculating the slope of a section of a rising edge of a scattering profile (line "C") and the slope of a falling edge of a scattering profile (line "E").

Any two points of the rising edge of a scattering profile or of the falling edge of a scattering profile may be selected when calculating the slope of the selected edge. According to one exemplary approach, the two points correspond to the individual light detectors that have generated signals which are one of two selected percentages of the signal at the apex "A" of the scattering profile. In one example, the two selected percentages are 35% of the signal at the apex of the scattering profile and 65% of the signal at the apex of the scattering profile. When analyzing the rising edge of a scattering profile, the leftmost of the two selected light detectors will be the one having a signal that is 35% of the signal at the apex of the scattering profile, with the rightmost of the two selected light detectors being the one having a signal that is 65% of the signal at the apex. When analyzing the falling edge of a scattering profile (as in Fig. 8), the leftmost of the two selected light detectors will be the one having a signal that is 65% of the signal at the apex, with the rightmost of the two selected light detectors being the one having a signal that is 35% of the signal at the apex.

According to another exemplary approach, the two points of the scattering profile selected for calculating the slope of the rising edge or the slope of the falling edge of the scattering profile correspond to the signals generated by two preselected or predetermined light detectors of the light detector array 104. When implementing this approach, the signals generated by the same two light detectors are used (e.g., always using the signal generated by the light detector having a detector position of 280 and always using the signal generated by the light detector having a detector position of 350), regardless of the maximum voltage of the signal and regardless of the percentage of that maximum voltage represented by the signals generated by each of the two light detectors. The exact two light detectors that are used may vary without departing from the scope of the present disclosure, though it may be advantageous to select two light detectors that are likely to both be generating signals located within the rising edge of the scattering profile or that are likely to both be generating signals located within the falling edge of the scattering profile, with there being sufficient separation between the positions of the two light detectors to provide an accurate approximation of the slope of the corresponding edge of the scattering profile.

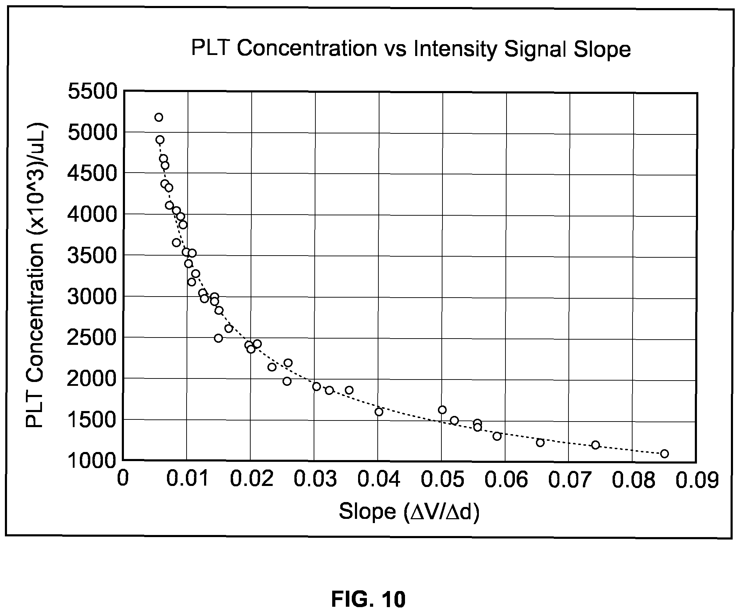

Regardless of the manner in which the rising edge slope or the falling edge slope is calculated, it has been found that the magnitude of the rising edge slope and the falling edge slope of a scattering profile are each indicative of the cellular concentration of a fluid being monitored by the optical detection assembly 100. A library or database of substance concentrations each correlated to a rising edge slope and/or a falling edge slope may be programmed into the controller or may be stored elsewhere and be remotely accessed by the controller. For example, Fig. 10 is a chart that illustrates an empirically derived correlation between rising edge slope or falling edge slope and the platelet concentration of a fluid. In the example shown in Fig. 8, the magnitude of the falling edge slope is approximately 0.023, which corresponds to a platelet concentration of approximately 2,250 × 10³ platelets / µL.

As a scattering profile will have both a rising edge and a falling edge, the slope of either may be calculated and compared to the library or database of correlations to determine the fluid characteristic that is correlated to the slope. A scattering profile will frequently be substantially symmetrical about its apex, such that the slope of a section of the rising edge will tend to be approximately equal to the slope of the corresponding section of the falling edge, in which case each slope will correlate to the same approximate fluid characteristic value. However, it is within the scope of the present disclosure for both slopes to be employed when determining a fluid characteristic value. According to one exemplary approach, the rising edge slope and the falling edge slope may both be calculated, with the controller then calculating the average of the two slopes and then comparing the average value to the library or database of correlations to determine the fluid characteristic value. According to another exemplary approach, the rising edge slope and the falling edge slope may both be calculated, with the controller comparing each slope to the library or database to determine the fluid characteristic value correlated to each slope. The controller may then calculate the average of those two fluid characteristic values, with the controller determining the fluid characteristic value of the subject fluid to be equal to the calculated average. Other approaches to employing the slopes of both the rising edge and the falling edge of a scattering profile to determine a fluid characteristic value (e.g., giving more weight to one of the slopes than the other) may also be employed without departing from the scope of the present disclosure.

According to another aspect of the present disclosure, an optical detection assembly may be used to analyze both a reference fluid having a known fluid characteristic value (e.g., a cellular concentration) and a subject fluid having an unknown value for the same fluid characteristic in order to determine the fluid characteristic value for the subject fluid. A subject fluid may have a composition that impacts optical analysis of the type described above, which may lead to an incorrect determination of the fluid characteristic value of interest. For example, plasma may contain proteins or lipids that affect its color or optical clarity. Lipids may cause light to scatter and distribute throughout a fluid in a similar manner to cells, such that optical analysis of lipemic plasma may overestimate the concentration of platelets in the plasma (due to light transmitted into the plasma being scattered by both the platelets and the lipids before reaching a light detector assembly). By analyzing a reference fluid, such effects may be accounted for, leading to a more accurate determination of the fluid characteristic value of interest (e.g., cellular concentration) for the subject fluid.

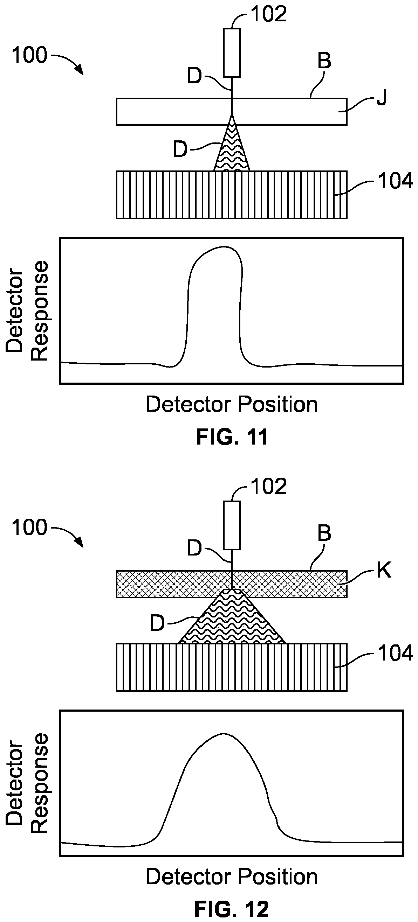

Figs. 11 and 12 illustrate an optical detection assembly 100 having a light source 102 and light detector array 104 of the type shown in Figs. 3-5, with the optical detection assembly 100 being used to analyze a reference fluid "J" (Fig. 11) and a subject fluid "K" (Fig. 12). While the principle of analyzing a reference fluid in order to improve the analysis of a subject fluid will be described with reference to an optical detection assembly employing a light detector array, it should be understood that it is not limited to use with such an optical detection assembly. Indeed, this principle may be employed with differently configured optical detection assemblies, including those having a single light detector, rather than a light detector array.

The reference fluid J is selected to have a composition (and, thus, optical characteristics) that is similar to the composition of the subject fluid K, except for the reference fluid J having a known value for the fluid characteristic of interest. As such, the nature of the reference fluid J may vary depending on the nature of the subject fluid K. In the case of the subject fluid K being platelet-rich plasma or platelet concentrate having an unknown platelet concentration that is to be determined using an optical detection assembly, the reference fluid J may be platelet-poor plasma or a supernatant that is substantially free of platelets (i.e., having a known platelet concentration of zero). This may be implemented using a biological fluid processing device 10 of the type shown in Fig. 1 during the course of a blood separation procedure of the type described above. More particularly, during such an exemplary procedure, blood is separated into packed red blood cells and platelet-rich plasma using the centrifugal separator 16, with the platelet-rich plasma being conveyed to the spinning membrane separator drive unit 14 via line L15, where the platelet-rich plasma is optically analyzed by the spinner inlet sensor M1. The platelet-rich plasma is separated into platelet concentrate and platelet-poor plasma (the supernatant) by the spinning membrane separator drive unit 14, with the platelet-poor plasma being conveyed out of the spinning membrane separator drive unit 14 via line L17, where it is optically analyzed by the spinner outlet sensor M2 (Fig. 2).

The signals generated by the spinner inlet sensor M1 and the spinner outlet sensor M2 are received by the controller 18, which compares the signals from the spinner inlet sensor M1 (which are indicative of the composition of the platelet-rich plasma) to the signals from the spinner outlet sensor M2 (which are indicative of the composition of the platelet-poor plasma or supernatant) in order to more accurately determine the platelet concentration of the platelet-rich plasma. This improvement is on account of the signals from the two sensors M1 and M2 being representative of two fluids that are identical (including any non-cellular components of the plasma that affect an optical analysis, such as lipids) except for their cellular composition, which is the fluid characteristic of interest. Otherwise, if the controller 18 were to use only the signals from the spinner inlet sensor M1 to determine the platelet concentration of the platelet-rich plasma, the result may be incorrect due to the effects of the presence of the various non-cellular components within the platelet-rich plasma on the optical analysis.

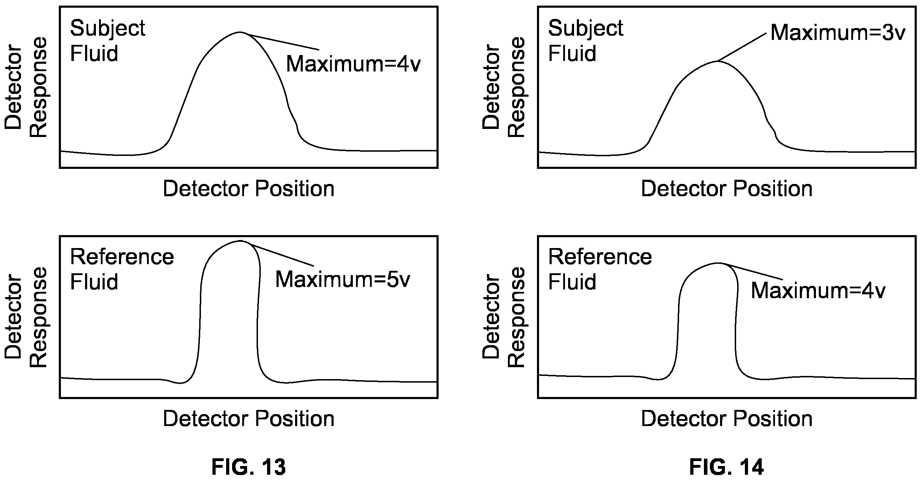

The nature of the comparison between the signals representing the composition of a reference fluid and the signals representing the composition of a subject fluid may vary without departing from the scope of the present disclosure. For example, when the signals are transmitted from a light detector assembly configured as a light detector array, a scatter profile may be generated by a controller, as described above. Fig. 13 illustrates the scattering profiles of a subject fluid (the upper chart) and a corresponding reference fluid (the lower chart). As described above, the rising edge slope and/or the falling edge slope of a scattering profile may be correlated to a fluid characteristic value (e.g., cellular concentration), such that a fluid characteristic value may be determined by the controller for each of the subject fluid and the reference fluid by comparing the rising edge slope and/or the falling edge slope to a library or database of correlations. The controller may then subtract the fluid characteristic value retrieved for the reference fluid from the fluid characteristic value retrieved for the subject fluid in order to arrive at a final or corrected value for the subject fluid.

According to an alternative approach, the maximum voltage of the signals from a light detection assembly may be indicative of the composition of a fluid, in which case the maximum voltage of a signal that is indicative of the composition of a reference fluid may be correlated to a fluid characteristic value by the controller. The controller may do the same for the maximum voltage of the signal representing the composition of a subject fluid, followed by the controller subtracting the fluid characteristic value of the reference fluid from the fluid characteristic value of the subject fluid in order to arrive at a final or corrected value for the subject fluid. Alternatively, the controller may subtract the maximum voltage of the signal representing the composition of the subject fluid from the maximum voltage of the signal representing the composition of the reference fluid, with the controller then accessing a library or database of values to correlate the voltage difference to a fluid characteristic value that constitutes a final or corrected value for the subject fluid. For example, Fig. 13 illustrates a subject fluid scattering profile having a maximum signal voltage of 4 V, with a reference fluid scattering profile having a maximum signal voltage of 5 V. The difference between the two maximum voltages is 1 V, which a controller may correlate to a final or corrected fluid characteristic value (e.g., cellular concentration) for the subject fluid.

A comparison of Figs. 13 and 14 will illustrate the effects of the composition of a fluid on optical analyzation and the way in which the principles described herein can be employed to isolate a fluid characteristic of interest. As described above, Fig. 13 illustrates a subject fluid scattering profile having a maximum signal voltage of 4 V, with a reference fluid scattering profile having a maximum signal voltage of 5 V. Fig. 14 illustrates the scattering profiles for a subject fluid (the upper chart) and a corresponding reference fluid (the lower chart), with the subject fluids of Figs. 13 and 14 having the same cellular concentration, but different fluid compositions (e.g., with the subject fluid of Fig. 14 being platelet-rich plasma having a greater lipid content than the sample of platelet-rich plasma represented in Fig. 13). While the subject fluids of Figs. 13 and 14 have the same cellular concentration, it will be seen that the maximum voltage of the subject fluid scattering profile in Fig. 14 is 3 V, compared to the maximum voltage of 4 V of the subject fluid scattering profile of Fig. 13. Directly comparing these two subject fluid scattering profiles to each other would lead to the incorrect conclusion that the fluids have different cellular concentrations. In contrast, considering each subject fluid scattering profile in combination with the scattering profile of its corresponding reference fluid will result in the same voltage difference of 1 V, which leads to the correct determination that the subject fluids of Figs. 13 and 14 have the same cellular composition.

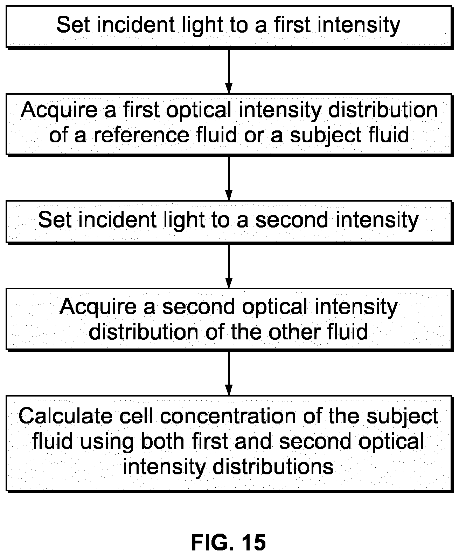

According to one embodiment, lights having different intensities may be used to analyze a subject fluid and the corresponding reference fluid. In the case of analysis of the cellular concentration of a fluid, a cell-free reference fluid does not contain any cells that significantly reduce the amount of transmitted light through the fluid, such that the relatively high intensity of light required to analyze a cell-containing fluid may be too high for the reference fluid, leading to saturated signals. Conversely, the intensity of light required to acquire a non-saturated signal for a cell-free reference fluid will typically be too low for analysis of a cell-containing subject fluid, leading to low or unmeasurable signal strengths. Therefore, it may be advantageous for the intensity of light used to analyze a reference fluid to be less than the intensity of the light used to analyze a subject fluid. An exemplary routine for practicing this principle is illustrated in Fig. 15.

Any method for achieving different intensity incident light may be employed without departing from the scope of the present disclosure. In one exemplary embodiment, a single light source 102 may be configured to selectively emit light having different intensity, with a lower intensity being used when analyzing a reference fluid J (as in Fig. 11) and a greater intensity being used when analyzing a subject fluid K (as in Fig. 12). In such an embodiment, a single optical detection assembly 100 and a single vessel B may be employed to analyze both the reference fluid J and the subject fluid K. In another exemplary embodiment, two separate, fixed-intensity light sources each configured to emit light having a different intensity may be employed in combination with separate fluid paths or vessels and separate optical detection assemblies (e.g., with a spinner inlet sensor M1 being provided with a single light source configured to emit light having a relatively high intensity and a spinner outlet sensor M2 being provided with a single light source configured to emit light having a lower intensity). Alternatively, rather than employing two optical detection assemblies each having one light source, a single optical detection assembly may be provided with a light source assembly comprising first and second light sources. In such an embodiment, one of the fluids is first analyzed by the optical detection assembly using the appropriate light source, followed by the other fluid being analyzed using the other light source. This may entail conveying the subject fluid and the reference fluid through the same vessel at different times during a procedure or employing a single optical detection assembly that is configured to analyze fluids in two different vessels (with one vessel being associated with one of the light sources and used for the flow of the subject fluid and the other vessel being associated with the other light source and used for the flow of the reference fluid).

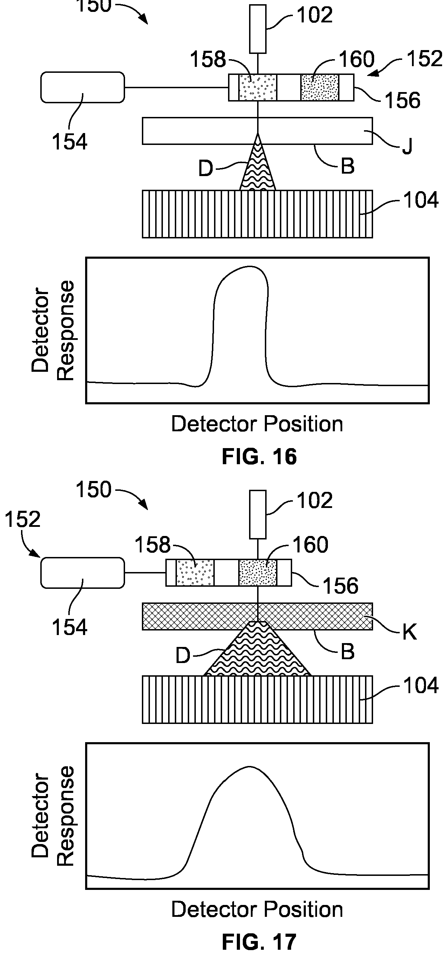

Figs. 16 and 17 illustrate an alternative embodiment of an optical detection assembly 150 that is suitable for analyzing a reference fluid J and a subject fluid K with lights having different intensities. In the embodiment of Figs. 16 and 17, the optical detection assembly 150 includes an optical filter assembly 152 comprising an actuator 154 (e.g., a linear actuator) and a carriage 156, in addition to including a single light source 102 (configured to emit light having a constant intensity) and light detector array 104 of the type previously described. At least one optical filter is incorporated into the carriage 156, with the optical filter reducing the intensity of light from the light B and the fluid present therein. In the illustrated embodiment, the carriage 156 has a first optical filter 158 and a second optical filter 160, with each optical filter being configured to allow light having a different intensity to reach the vessel B and the fluid present therein.

In such an embodiment, the actuator 154 is controlled by a controller to move the appropriate optical filter into the path of the light from the light source 102, depending on which fluid is present in the vessel B. If the carriage 156 includes two optical filters (as in Figs. 16 and 17), the controller will command the actuator 154 to move the carriage 156 so as to place the more light-restrictive optical filter 158 into alignment with the light source 102 when a reference fluid J is present in the vessel B as in Fig. 16). When the subject fluid K is present in the vessel B and light having a greater intensity is appropriate, the controller will command the actuator 154 to move the carriage 156 so as to place the less light-restrictive optical filter 160 into alignment with the light source 102 (as in Fig. 17). When the carriage 156 includes only one optical filter, the controller will command the actuator 154 to move the carriage 156 so as to place the single optical filter into alignment with the light source 102 when the reference fluid J is present in the vessel B, with the controller moving the optical filter out of alignment with the light source 102 (e.g., to align an aperture or opening defined by the carriage 156 into alignment with the light source 102) when a subject fluid K is present within the vessel B so as to allow for the subject fluid to be analyzed with light having a greater intensity.

While it may be advantageous for some optical detection assemblies to apply light having different intensities when analyzing a reference fluid and a subject fluid, it should be understood that some light detectors may be capable of receiving greater amounts of light without becoming saturated. Thus, when such light detector assemblies are employed, the same intensity of incident light may be used for analyzing both a reference fluid and a subject fluid.

### V. Determination Of PMI

As described above, data may be extracted from a scattering profile to determine various characteristics of a subject fluid. For example, in addition to the cellular concentration of the fluid, another one of the characteristics of a platelet-containing fluid that may be determined by analyzing a scattering profile is the PMI of the fluid.

Just as Figs. 6 and 7 respectively illustrate fluids having relatively low and relatively high concentrations of platelets, they illustrate fluids having a relatively low PMI (Fig. 6) and a relatively high PMI (Fig. 7). In accordance with the above description of the scattering profiles generated for fluids having different platelet concentrations, the scattering profiles generated for fluids having different PMIs will be differently shaped, with different maximum signals, different rising edge slopes, and different falling edge slopes. More particularly, in the illustrated embodiment, platelets within a fluid cause light to scatter, rather than being transmitted straight through the fluid and vessel B (along its initial path). As there is a greater volume of platelets in the fluid F of Fig. 7, there is more scattering of the light, with more individual light detectors receiving at least some of the light, though with a relatively low maximum intensity compared to the maximum intensity of the light received by an individual light detector in Fig. 6. Stated differently, light passing through the lower PMI fluid f of Fig. 6 is narrowly distributed or dispersed, while light passing through the higher PMI fluid F of Fig. 7 is more widely or broadly distributed or dispersed. Thus, by providing a light detector array 104, the intensity of light received by multiple individual light detectors (i.e., the light distribution or the scattering profile) may be assessed to determine the PMI of a substance.

In accordance with the above description of the determination of the cellular concentration of a fluid, once the controller has generated a scattering profile, it may employ various approaches to extract data from the scattering profile that can be used to determine the PMI of the subject fluid. In particular, it has been found that the slope of the rising edge of a scattering profile and/or the slope of the falling edge of a scattering profile is indicative of the PMI of a fluid.

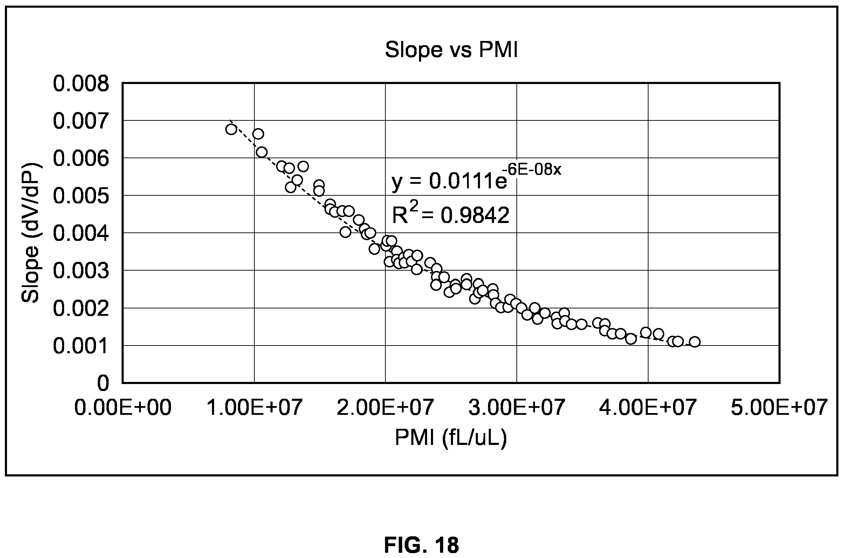

The controller may employ different techniques to calculate the slope of a section of the rising edge of a scattering profile or to calculate the slope of a section of the falling edge of a scattering profile, with the same techniques described above in the description of the determination of the cellular concentration of a fluid being equally applicable to determination of the PMI of a fluid. A library or database of PMIs each correlated to a rising edge slope and/or a falling edge slope may be programmed into the controller or may be stored elsewhere and be remotely accessed by the controller. For example, Fig. 18 is a chart that illustrates an empirically derived correlation between rising edge slope or falling edge slope and the PMI of a fluid, with the chart being created using data from a cell counter of the type marketed by the Sysmex Corporation of Kobe, Japan. Once the controller has calculated the relevant slope, the controller may consult the library or database to correlate the slope to the PMI of the fluid being monitored.

In accordance with the above description of the determination of the cellular concentration of a fluid, as a scattering profile will have both a rising edge and a falling edge, the slope of either may be calculated and compared to the library or database of correlations to determine the PMI that is correlated to the slope. A scattering profile will frequently be substantially symmetrical about its apex, such that the slope of a section of the rising edge will tend to be approximately equal to the slope of the corresponding section of the falling edge, in which case each slope will correlate to the same approximate PMI. However, it is within the scope of the present disclosure for both slopes to be employed when determining the PMI of a substance. According to one exemplary approach, the rising edge slope and the falling edge slope may both be calculated, with the controller then calculating the average of the two slopes and then comparing the average value to the library or database of correlations to determine the PMI. According to another exemplary approach, the rising edge slope and the falling edge slope may both be calculated, with the controller comparing each slope to the library or database to determine the PMI correlated to each slope. The controller may then calculate the average of those two PMI values, with the controller determining the PMI of the subject fluid to be equal to the calculated average. Other approaches to employing the slopes of both the rising edge and the falling edge of a scattering profile to determine a PMI (e.g., giving more weight to one of the slopes than the other) may also be employed without departing from the scope of the present disclosure.

Once the PMI of a substance has been determined, it may be used by the controller in any of a number of ways. For example, as described above, the PMI of a substance may be used as a factor in determining the appropriate volume of a storage solution to be added to the substance. In another example, if the controller has also determined the platelet concentration of the substance (according to the approach described above or using a different optical detection assembly or according to any other suitable approach), the controller may calculate the MPV of the substance by dividing the PMI by the platelet concentration. If the MPV is unusually high or low (which may be indicative of a number of different diseases or disorders), the controller may generate an alert or add an appropriate note to the profile of the blood source.

### VI. Aspects

Aspect 1. An optical detection assembly for monitoring a fluid in a vessel, comprising: a light source configured and oriented to emit a light into a fluid in a vessel; a light detector array comprising a plurality of light detectors and configured to receive at least a portion of the light exiting the vessel; and a controller programmed to receive signals from the light detector array indicative of an intensity of said at least a portion of the light received by each one of said plurality of light detectors, generate a scattering profile based at least in part on said signals from the light detector array, with the scattering profile including a rising edge and a falling edge, calculate a slope of the rising edge or the falling edge of the scattering profile, and determine a platelet mass index and/or concentration of a substance in the fluid in the vessel based at least in part on said slope.

Aspect 2. The optical detection assembly of Aspect 1, wherein a library of substance concentrations and/or a library of platelet mass indices each correlated to a different rising edge slope and/or falling edge slope is programmed into the controller.

Aspect 3. The optical detection assembly of Aspect 1, wherein the controller is programmed to remotely access a library of substance concentrations and/or a library of platelet mass indices each correlated to a different rising edge slope and/or falling edge slope.

Aspect 4. The optical detection assembly of any one of the preceding Aspects, wherein the controller is programmed to determine the platelet mass index and/or the concentration of the substance in the fluid in the vessel based on the rising edge slope and not on the falling edge slope or based on the falling edge slope and not on the rising edge slope.

Aspect 5. The optical detection assembly of any one of Aspects 1-3, wherein the controller is programmed to determine the platelet mass index and/or the concentration of the substance in the fluid in the vessel based on both the rising edge slope and the falling edge slope.

Aspect 6. The optical detection assembly of any one of the preceding Aspects, wherein the controller is programmed to calculate the rising edge slope or the falling edge slope by dividing a difference between a first voltage of a first signal from a first light detector and a second voltage from a second light detector by a difference between the relative positions of the first and second light detectors within the light detector array.

Aspect 7. The optical detection assembly of Aspect 6, wherein the first and second light detectors are predetermined or preselected based on the positions of the first and second light detectors within the light detector array.

Aspect 8. The optical detection assembly of Aspect 6, wherein the first voltage is a first percentage of a maximum voltage of the signals received by the controller from the light detector array, and the second voltage is a second percentage of the maximum voltage of the signals received by the controller from the light detector array.

Aspect 9. The optical detection assembly of Aspect 8, wherein one of the first and second voltages is 35% of the maximum voltage, and the other one of the first and second voltages is 65% of the maximum voltage.

Aspect 10. The optical detection assembly of any one of Aspects 1-6, wherein the controller is programmed to employ a linear regression to calculate the rising edge slope or the falling edge slope.

Aspect 11. A biological fluid processing device comprising: a pump system; a valve system; a controller programmed to control the operation of the pump system and the valve system to execute a biological fluid processing procedure; and an optical detection assembly comprising a light source configured and oriented to emit a light into a fluid in a vessel, and a light detector array comprising a plurality of light detectors and configured to receive at least a portion of the light exiting the vessel, wherein the controller is further programmed to receive signals from the light detector array indicative of an intensity of said at least a portion of the light received by each one of said plurality of light detectors, generate a scattering profile based at least in part on said signals from the light detector array, with the scattering profile including a rising edge and a falling edge, calculate a slope of the rising edge or the falling edge of the scattering profile, and determine a platelet mass index and/or a concentration of a substance in the fluid in the vessel based at least in part on said slope.

Aspect 12. The biological fluid processing device of Aspect 11, wherein a library of substance concentrations and/or a library of platelet mass indices each correlated to a different rising edge slope and/or falling edge slope is programmed into the controller.

Aspect 13. The biological fluid processing device of Aspect 11, wherein the controller is programmed to remotely access a library of substance concentrations and/or a library of platelet mass indices each correlated to a different rising edge slope and/or falling edge slope.

Aspect 14. The biological fluid processing device of any one of Aspects 11-13, wherein the controller is programmed to determine the platelet mass index and/or the concentration of the substance in the fluid in the vessel based on the rising edge slope and not on the falling edge slope or based on the falling edge slope and not on the rising edge slope.

Aspect 15. The biological fluid processing device of any one of Aspects 11-13, wherein the controller is programmed to determine the platelet mass index and/or the concentration of the substance in the fluid in the vessel based on both the rising edge slope and the falling edge slope.

Aspect 16. The biological fluid processing device of any one of Aspects 11-16, wherein the controller is programmed to calculate the rising edge slope or the falling edge slope by dividing a difference between a first voltage of a first signal from a first light detector and a second voltage from a second light detector by a difference between the relative positions of the first and second light detectors within the light detector array.

Aspect 17. The biological fluid processing device of Aspect 16, wherein the first and second light detectors are predetermined or preselected based on the positions of the first and second light detectors within the light detector array.

Aspect 18. The biological fluid processing device of Aspect 16, wherein the first voltage is a first percentage of a maximum voltage of the signals received by the controller from the light detector array, and the second voltage is a second percentage of the maximum voltage of the signals received by the controller from the light detector array.

Aspect 19. The biological fluid processing device of Aspect 18, wherein one of the first and second voltages is 35% of the maximum voltage, and the other one of the first and second voltages is 65% of the maximum voltage.

Aspect 20. The biological fluid processing device of any one of Aspects 11-16, wherein the controller is programmed to employ a linear regression to calculate the rising edge slope or the falling edge slope.

Aspect 21. A method of determining a platelet mass index and/or a concentration of a substance in a fluid in a vessel comprising: emitting a light into a fluid in a vessel; receiving at least a portion of the light exiting the vessel with a plurality of light detectors of a light detector array; generating a scattering profile based at least in part on an intensity of said at least a portion of the light received by each one of said plurality of light detectors, with the scattering profile including a rising edge and a falling edge; calculating a slope of the rising edge or the falling edge of the scattering profile; and determining a platelet mass index and/or a concentration of a substance in the fluid in the vessel based at least in part on said slope.

Aspect 22. The method of Aspect 21, wherein said determining the platelet mass index and/or the concentration of the substance in the fluid in the vessel includes accessing a local library of platelet mass indices and/or a local library of substance concentrations each correlated to a different rising edge slope and/or falling edge slope.

Aspect 23. The method of Aspect 21, wherein said determining the platelet mass index and/or the concentration of the substance in the fluid in the vessel includes accessing a remote library of platelet mass indices and/or a remote library of substance concentrations each correlated to a different rising edge slope and/or falling edge slope.

Aspect 24. The method of any one of Aspects 21-23, wherein the determination of the platelet mass index and/or the concentration of the substance in the fluid in the vessel is based on the rising edge slope and not on the falling edge slope or based on the falling edge slope and not on the rising edge slope.

Aspect 25. The method of any one of Aspects 21-23, wherein the determination of the concentration of the substance in the fluid in the vessel is based on both the rising edge slope and the falling edge slope.

Aspect 26. The method of any one of Aspects 21-25, wherein the rising edge slope or the falling edge slope is calculated by dividing a difference between a first voltage of a first signal from a first light detector and a second voltage from a second light detector by a difference between the relative positions of the first and second light detectors within the light detector array.

Aspect 27. The method of Aspect 26, wherein the first and second light detectors are predetermined or preselected based on the positions of the first and second light detectors within the light detector array.

Aspect 28. The method of Aspect 26, wherein the first voltage is a first percentage of a maximum voltage of signals from the light detector array comprising the scattering profile, and the second voltage is a second percentage of the maximum voltage of the signals from the light detector array comprising the scattering profile.

Aspect 29. The method of Aspect 28, wherein one of the first and second voltages is 35% of the maximum voltage, and the other one of the first and second voltages is 65% of the maximum voltage.

Aspect 30. The method of any one of Aspects 21-26, wherein the rising edge slope or the falling edge slope is calculated using a linear regression.

Aspect 31. An optical detection assembly for monitoring a fluid in a vessel, comprising: a light source assembly configured and oriented to emit a light into a fluid in a vessel; a light detector assembly configured to receive at least a portion of the light exiting the vessel; and a controller programmed to receive a first signal from the light detector assembly indicative of an intensity of said at least a portion of the light received by the light detector assembly when a reference fluid is in the vessel, receive a second signal from the light detector assembly indicative of an intensity of said at least a portion of the light received by the light detector assembly when a subject fluid is in the vessel, compare the first signal and the second signal, and determine a concentration of a substance in the subject fluid in the vessel based at least in part on the comparison between the first signal and the second signal.

Aspect 32. The optical detection assembly of Aspect 31, wherein the reference fluid has a known concentration of said substance.

Aspect 33. The optical detection assembly of any one of Aspects 31-32, wherein the reference fluid is free of said substance.

Aspect 34. The optical detection assembly of any one of Aspects 31-33, wherein the reference fluid comprises platelet-poor plasma or a supernatant, the subject fluid comprises platelet-rich plasma or platelet concentrate, and the substance comprises platelets.

Aspect 35. The optical detection assembly of any one of Aspects 31-34, wherein the light entering into the reference fluid has a first intensity, the light entering into the subject fluid has a second intensity, and the first intensity is different from the second intensity.

Aspect 36. The optical detection assembly of Aspect 35, wherein the concentration of the substance in the reference fluid is less than the concentration of the substance in the subject fluid, and the second intensity is greater than the first intensity.

Aspect 37. The optical detection assembly of any one of Aspects 31-36, wherein the light source assembly comprises a first light source and a second light source, the controller is programmed to control the first light source to emit light when the reference fluid is in the vessel, and the controller is programmed to control the second light source to emit light when the subject fluid is in the vessel.

Aspect 38. The optical detection assembly of any one of Aspects 31-36, wherein the light source assembly comprises a single light source, and the controller is programmed to control the single light source to emit lights having different intensities.

Aspect 39. The optical detection assembly of any one of Aspects 31-36, wherein the light source assembly comprises a single light source, the optical detection assembly further includes an optical filter assembly comprising an optical filter incorporated into a carriage and an actuator associated with the carriage, and the controller is programmed to control the actuator to move the carriage so as to selectively place the optical filter into and out of a path of the light emitted by the light source assembly.

Aspect 40. The optical detection assembly of Aspect 39, wherein the optical filter assembly includes a first optical filter and a second optical filter, the controller is programmed to control the actuator to move the carriage so as to place the first optical filter into the path of the light emitted by the light source assembly when the reference fluid is in the vessel, and the controller is programmed to control the actuator to move the carriage so as to place the second optical filter into the path of the light emitted by the light source assembly when the subject fluid is in the vessel.

Aspect 41. The optical detection assembly of any one of Aspects 31-40, wherein the light detector assembly comprises a light detector array including a plurality of light detectors, the first signal is indicative of the intensity of said at least a portion of the light received by each one of said plurality of light detectors when the reference fluid is in the vessel, the second signal is indicative of the intensity of said at least a portion of the light received by each one of said plurality of light detectors when the subject fluid is in the vessel, and the controller is programmed to generate a first scattering profile based at least in part on the first signal, generate a second scattering profile based at least in part on the second signal, and determine the concentration of the substance in the subject fluid in the vessel based at least in part on a comparison between the first scattering profile and the second scattering profile.

Aspect 42. The optical detection assembly of any one of Aspects 31-41, wherein the controller is programmed to compare a difference between voltages of the first signal and the second signal to determine the concentration of the substance in the subject fluid in the vessel.

Aspect 43. The optical detection assembly of Aspect 41, wherein the controller is programmed to calculate a first slope of a rising edge of the first scattering profile, calculate a second slope of a rising edge of the second scattering profile, and determine the concentration of the substance in the subject fluid in the vessel based at least in part on a comparison of the first slope to the second slope.

Aspect 44. The optical detection assembly of Aspect 41, wherein the controller is programmed to calculate a first slope of a falling edge of the first scattering profile, calculate a second slope of a falling edge of the second scattering profile, and determine the concentration of the substance in the subject fluid in the vessel based at least in part on a comparison of the first slope to the second slope.

Aspect 45. The optical detection assembly of Aspect 41, wherein the controller is programmed to calculate a first rising edge slope and a first falling edge slope of the first scattering profile, calculate a second rising edge slope and a second falling edge slope of the second scattering profile, and determine the concentration of the substance in the subject fluid in the vessel based at least in part on a comparison of the first rising edge slope to the second rising edge slope and a comparison of the first falling edge slope to the second falling edge slope.

Aspect 46. A biological fluid processing device comprising: a pump system; a valve system; a controller programmed to control the operation of the pump system and the valve system to execute a biological fluid processing procedure; and an optical detection assembly comprising a light source assembly configured and oriented to emit a light into a fluid in a vessel, and a light detector assembly configured to receive at least a portion of the light exiting the vessel, wherein the controller is further programmed to receive a first signal from the light detector assembly indicative of an intensity of said at least a portion of the light received by the light detector assembly when a reference fluid is in the vessel, receive a second signal from the light detector assembly indicative of an intensity of said at least a portion of the light received by the light detector assembly when a subject fluid is in the vessel, compare the first signal and the second signal, and determine a concentration of a substance in the subject fluid in the vessel based at least in part on the comparison between the first signal and the second signal.

Aspect 47. The biological fluid processing device of Aspect 46, wherein the reference fluid has a known concentration of said substance.

Aspect 48. The biological fluid processing device of any one of Aspects 46-47, wherein the reference fluid is free of said substance.

Aspect 49. The biological fluid processing device of any one of Aspects 46-48, wherein the reference fluid comprises platelet-poor plasma or a supernatant, the subject fluid comprises platelet-rich plasma or platelet concentrate, and the substance comprises platelets.

Aspect 50. The biological fluid processing device of any one of Aspects 46-49, wherein the light entering into the reference fluid has a first intensity, the light entering into the subject fluid has a second intensity, and the first intensity is different from the second intensity.

Aspect 51. The biological fluid processing device of Aspect 50, wherein the concentration of the substance in the reference fluid is less than the concentration of the substance in the subject fluid, and the second intensity is greater than the first intensity.

Aspect 52. The biological fluid processing device of any one of Aspects 46-51, wherein the light source assembly comprises a first light source and a second light source, the controller is programmed to control the first light source to emit light when the reference fluid is in the vessel, and the controller is programmed to control the second light source to emit light when the subject fluid is in the vessel.

Aspect 53. The biological fluid processing device of any one of Aspects 46-51, wherein the light source assembly comprises a single light source, and the controller is programmed to control the single light source to emit lights having different intensities.

Aspect 54. The biological fluid processing device of any one of Aspects 46-51, wherein the light source assembly comprises a single light source, the optical detection assembly further includes an optical filter assembly comprising an optical filter incorporated into a carriage and an actuator associated with the carriage, and the controller is programmed to control the actuator to move the carriage so as to selectively place the optical filter into and out of a path of the light emitted by the light source assembly.

Aspect 55. The biological fluid processing device of Aspect 54, wherein the optical filter assembly includes a first optical filter and a second optical filter, the controller is programmed to control the actuator to move the carriage so as to place the first optical filter into the path of the light emitted by the light source assembly when the reference fluid is in the vessel, and the controller is programmed to control the actuator to move the carriage so as to place the second optical filter into the path of the light emitted by the light source assembly when the subject fluid is in the vessel.

Aspect 56. The biological fluid processing device of any one of Aspects 46-55, wherein the light detector assembly comprises a light detector array including a plurality of light detectors, the first signal is indicative of the intensity of said at least a portion of the light received by each one of said plurality of light detectors when the reference fluid is in the vessel, the second signal is indicative of the intensity of said at least a portion of the light received by each one of said plurality of light detectors when the subject fluid is in the vessel, and the controller is programmed to generate a first scattering profile based at least in part on the first signal, generate a second scattering profile based at least in part on the second signal, and determine the concentration of the substance in the subject fluid in the vessel based at least in part on a comparison between the first scattering profile and the second scattering profile.

Aspect 57. The biological fluid processing device of any one of Aspects 46-56, wherein the controller is programmed to compare a difference between voltages of the first signal and the second signal to determine the concentration of the substance in the subject fluid in the vessel.

Aspect 58. The biological fluid processing device of Aspect 56, wherein the controller is programmed to calculate a first slope of a rising edge of the first scattering profile, calculate a second slope of a rising edge of the second scattering profile, and determine the concentration of the substance in the subject fluid in the vessel based at least in part on a comparison of the first slope to the second slope.

Aspect 59. The biological fluid processing device of Aspect 56, wherein the controller is programmed to calculate a first slope of a falling edge of the first scattering profile, calculate a second slope of a falling edge of the second scattering profile, and determine the concentration of the substance in the subject fluid in the vessel based at least in part on a comparison of the first slope to the second slope.

Aspect 60. The biological fluid processing device of Aspect 56, wherein the controller is programmed to calculate a first rising edge slope and a first falling edge slope of the first scattering profile, calculate a second rising edge slope and a second falling edge slope of the second scattering profile, and determine the concentration of the substance in the subject fluid in the vessel based at least in part on a comparison of the first rising edge slope to the second rising edge slope and a comparison of the first falling edge slope to the second falling edge slope.

Aspect 61. A method of determining a concentration of a substance in a subject fluid in a vessel comprising: emitting a first light into a reference fluid in a vessel; receiving at least a portion of the first light exiting the vessel with a light detector assembly; generating a first signal indicative of an intensity of said at least a portion of the first light received by the light detector assembly; emitting a second light into a subject fluid in the vessel; receiving at least a portion of the second light exiting the vessel with the light detector assembly; generating a second signal indicative of an intensity of said at least a portion of the second light received by the light detector assembly; comparing the first signal to the second signal; and determining a concentration of a substance in the subject fluid in the vessel based at least in part on the comparison between the first signal and the second signal.

Aspect 62. The method of Aspect 61, wherein the reference fluid has a known concentration of said substance.

Aspect 63. The method of any one of Aspects 61-62, wherein the reference fluid is free of said substance.

Aspect 64. The method of any one of Aspects 61-63, wherein the reference fluid comprises platelet-poor plasma or a supernatant, the subject fluid comprises platelet-rich plasma or platelet concentrate, and the substance comprises platelets.

Aspect 65. The method of any one of Aspects 61-64, wherein the light entering into the reference fluid has a first intensity, the light entering into the subject fluid has a second intensity, and the first intensity is different from the second intensity.

Aspect 66. The method of Aspect 65, wherein the concentration of the substance in the reference fluid is less than the concentration of the substance in the subject fluid, and the second intensity is greater than the first intensity.

Aspect 67. The method of any one of Aspects 61-66, wherein the first light is emitted by a first light source and the second light is emitted by a second light source.

Aspect 68. The method of any one of Aspects 61-66, wherein the first and second lights have different intensities and are emitted by a single light source.

Aspect 69. The method of any one of Aspects 61-66, wherein the first and second lights are emitted by a single light source, and at least one of said emitting the first light and said emitting the second light includes moving an optical filter into a path of the light emitted by the single light source.

Aspect 70. The method of Aspect 69, wherein said emitting the first light includes moving a first optical filter into a path of the first light, and said emitting the second light includes moving a second optical filter into a path of the second light.

Aspect 71. The method of any one of Aspects 61-70, wherein said portions of the first and second lights are received by a light detector array including a plurality of light detectors, the first signal is indicative of the intensity of said at least a portion of the first light received by each one of said plurality of light detectors, the second signal is indicative of the intensity of said at least a portion of the second light received by each one of said plurality of light detectors, and said comparing the first signal to the second signal includes generating a first scattering profile based at least in part on the first signal, generating a second scattering profile based at least in part on the second signal, and comparing the first scattering profile and the second scattering profile.

Aspect 72. The method of any one of Aspects 61-71, wherein said comparing the first signal to the second signal includes comparing a difference between voltages of the first signal and the second signal.

Aspect 73. The method of Aspect 71, said comparing the first scattering profile and the second scattering profile includes calculating a first slope of a rising edge of the first scattering profile, calculating a second slope of a rising edge of the second scattering profile, and comparing the first slope to the second slope.

Aspect 74. The method of Aspect 71, wherein said comparing the first scattering profile and the second scattering profile includes calculating a first slope of a falling edge of the first scattering profile, calculating a second slope of a falling edge of the second scattering profile, and comparing the first slope to the second slope.

Aspect 75. The method of Aspect 71, wherein said comparing the first scattering profile and the second scattering profile includes calculating a first rising edge slope and a first falling edge slope of the first scattering profile, calculating a second rising edge slope and a second falling edge slope of the second scattering profile, comparing the first rising edge slope to the second rising edge slope, and comparing the first falling edge slope to the second falling edge slope.

It will be understood that the embodiments described above are illustrative of some of the applications of the principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

## Claims

1. An optical detection assembly (100) for monitoring a fluid in a vessel (B), comprising:
a light source (102) configured and oriented to emit a light (D) into a fluid in a vessel (B);
a light detector array (104) comprising a plurality of light detectors and configured to receive at least a portion of the light (D) exiting the vessel (B); and
a controller (18) programmed to
receive signals from the light detector array (104) indicative of an intensity of said at least a portion of the light (D) received by each one of said plurality of light detectors,
generate a scattering profile based at least in part on said signals from the light detector array (104), with the scattering profile including a rising edge and a falling edge,
calculate a slope of the rising edge or the falling edge of the scattering profile, and
determine a platelet mass index and/or a concentration of a substance in the fluid in the vessel (B) based at least in part on said slope.

2. The optical detection assembly (100) of claim 1, wherein a library of substance concentrations and/or a library of platelet mass indices each correlated to a different rising edge slope and/or falling edge slope is programmed into the controller (18).

3. The optical detection assembly (100) of claim 1, wherein the controller (18) is programmed to remotely access a library of substance concentrations and/or a library of platelet mass indices each correlated to a different rising edge slope and/or falling edge slope.

4. The optical detection assembly (100) of any one of the preceding claims, wherein the controller (18) is programmed to determine the platelet mass index and/or the concentration of the substance in the fluid in the vessel (B) based on the rising edge slope and not on the falling edge slope or based on the falling edge slope and not on the rising edge slope.

5. The optical detection assembly (100) of any one of claims 1-3, wherein the controller (18) is programmed to determine the platelet mass index and/or the concentration of the substance in the fluid in the vessel (B) based on both the rising edge slope and the falling edge slope.

6. The optical detection assembly (100) of any one of the preceding claims, wherein the controller (18) is programmed to calculate the rising edge slope or the falling edge slope by dividing a difference between a first voltage of a first signal from a first light detector and a second voltage from a second light detector by a difference between the relative positions of the first and second light detectors within the light detector array (104).

7. The optical detection assembly (100) of claim 6, wherein the first and second light detectors are predetermined or preselected based on the positions of the first and second light detectors within the light detector array (104).

8. The optical detection assembly (100) of claim 6, wherein
the first voltage is a first percentage of a maximum voltage of the signals received by the controller (18) from the light detector array (104), and
the second voltage is a second percentage of the maximum voltage of the signals received by the controller (18) from the light detector array (104).

9. The optical detection assembly (100) of claim 8, wherein
one of the first and second voltages is 35% of the maximum voltage, and
the other one of the first and second voltages is 65% of the maximum voltage.

10. The optical detection assembly (100) of any one of claims 1-6, wherein the controller (18) is programmed to employ a linear regression to calculate the rising edge slope or the falling edge slope.

11. A biological fluid processing device (10) comprising:
a pump system (P1-P6);
a valve system (V1-V11); and
the optical detection assembly (100) of any one of the preceding claims, wherein the controller (18) is programmed to control the operation of the pump system (P1-P6) and the valve system (V1-V11) to execute a biological fluid processing procedure.

12. An optical detection assembly (100, 150) for monitoring a fluid in a vessel (B), comprising:
a light source assembly (102) configured and oriented to emit a light (D) into a fluid in a vessel (B);
a light detector assembly (104) configured to receive at least a portion of the light (D) exiting the vessel (B); and
a controller (18) programmed to
receive a first signal from the light detector assembly (104) indicative of an intensity of said at least a portion of the light (D) received by the light detector assembly (104) when a reference fluid is in the vessel (B),
receive a second signal from the light detector assembly (104) indicative of an intensity of said at least a portion of the light (D) received by the light detector assembly (104) when a subject fluid is in the vessel (B),
compare the first signal and the second signal, and
determine a concentration of a substance in the subject fluid in the vessel (B) based at least in part on the comparison between the first signal and the second signal.

13. The optical detection assembly (100, 150) of claim 12, wherein
the light (D) entering into the reference fluid has a first intensity,
the light (D) entering into the subject fluid has a second intensity, and
the first intensity is different from the second intensity.

14. The optical detection assembly (100, 150) of any one of claims 12-13, wherein
the light detector assembly (104) comprises a light detector array including a plurality of light detectors,
the first signal is indicative of the intensity of said at least a portion of the light (D) received by each one of said plurality of light detectors when the reference fluid is in the vessel (B),
the second signal is indicative of the intensity of said at least a portion of the light (D) received by each one of said plurality of light detectors when the subject fluid is in the vessel (B), and
the controller (18) is programmed to
generate a first scattering profile based at least in part on the first signal,
generate a second scattering profile based at least in part on the second signal, and
determine the concentration of the substance in the subject fluid in the vessel (B) based at least in part on a comparison between the first scattering profile and the second scattering profile.

15. The optical detection assembly (100, 150) of claim 14, wherein the controller (18) is programmed to
calculate a first rising edge slope and/or a first falling edge slope of the first scattering profile,
calculate a second rising edge slope and/or a second falling edge slope of the second scattering profile, and
determine the concentration of the substance in the subject fluid in the vessel (B) based at least in part on a comparison of the first rising edge slope to the second rising edge slope and/or a comparison of the first falling edge slope to the second falling edge slope.
